# FASCICULE DE BREVET EUROPEEN

(11) **EP 1 501 842 B1**
(45) Date de publication et mention de la délivrance du brevet: **09.05.2012**
(21) Numéro de dépôt: 03747145.5
(22) Date de dépôt: 17.04.2003
(51) Int. Cl.: C07F 9/6581

(54) **SUPPORTS SOLIDES FONCTIONNALISES PAR DES DENDRIMERES PHOSPHORES, LEUR PROCEDE DE PREPARATION ET APPLICATIONS**
FESTE, MIT PHOSPHORDENDRIMEREN FUNKTIONALISIERTE TRÄGER, VERFAHREN ZU DEREN HERSTELLUNG UND DEREN VERWENDUNGEN
SOLID SUPPORTS FUNCTIONALISED BY PHOSPHORUS DENDRIMERS, METHOD FOR PREPARING SAME AND USES THEREOF

(30) Priorité: 23.04.2002 FR 0205049
(43) Date de publication de la demande: 02.02.2005
(73) Titulaire: CENTRE NATIONAL DE LA RECHERCHE SCIENTIFIQUE, 75016 Paris (FR); INSTITUT NATIONAL DE LA RECHERCHE AGRONOMIQUE, 75007 Paris (FR); INSTITUT NATIONAL DES SCIENCES APPLIQUEES DE TOULOUSE, 31077 Toulouse Cedex 4 (FR)
(72) Inventeur: TREVISIOL, Emmanuelle, F-81500 Montcabrier (FR); LECLAIRE, Julien, 69008 Lyon (FR); PRATVIEL, Geneviève, F-31100 Toulouse (FR); CAMINADE, Anne-Marie, F-31400 Toulouse (FR); FRANCOIS, Jean, F-31320 Castenet Tolosan (FR); MAJORAL, Jean-Pierre, F-31520 Ramonville (FR); MEUNIER, Bernard, F-31320 Castenet (FR)
(74) Mandataire: Orès, Bernard
(86) Numéro de dépôt international: PCT/FR2003/001231
(87) Numéro de publication internationale: WO 2003/091304

(56) Documents cités:
- WO-A-00/55627
- WO-A-01/51689
- FR-A- 2 801 592
- TURRIN CEDRIC-OLIVIER ET AL: "Organic-Inorganic Hybrid Materials Incorporating Phosphorus-Containing Dendrimers" CHEMISTRY OF MATERIALS, AMERICAN CHEMICAL SOCIETY, WASHINGTON, US, vol. 12, no. 12, 2000, pages 3848-3856, XP002213478 ISSN: 0897-4756
- SOLER-ILLIA ET AL.: "New mesotextured hybrid materials made from assemblies of dendrimers and titanium (IV)-oxo-organo clusters" ANGEW. CHEM. INT. ED., vol. 39, no. 23, 2000, pages 4250-4254, XP002228108
- MARAVAL ET AL: "Varying topology of dendrimers - a new approach toward the synthesis of di-block dendrimers" EUR. J. INORG. CHEM., 2001, pages 1681-1691, XP002228109
- BENTERS R. ET AL.: "Dendrimer-activated solid supports for nucleic acid and protein microarrays" CHEMBIOCHEM, vol. 2, 2001, pages 686-694, XP002228110 cité dans la demande
- MARAVAL V: "Rapid synthesis of phosphorus-containing dendrimers with controlled molecular architectures: first example of surface-block, layer-block, and segment-block dendrimers issued from the same dendron" JOURNAL OF THE AMERICAN CHEMICAL SOCIETY, AMERICAN CHEMICAL SOCIETY, WASHINGTON, DC, US, vol. 122, no. 11, 22 mars 2000 (2000-03-22), pages 2499-2511, XP002144561 ISSN: 0002-7863
- GALLIOT C ET AL: "REGIOSELECTIVE STEPWISE GROWTH OF DENDRIMER UNITS IN THE INTERNAL VOIDS OF A MAIN DENDRIMER" SCIENCE, AMERICAN ASSOCIATION FOR THE ADVANCEMENT OF SCIENCE,, US, vol. 277, 26 septembre 1997 (1997-09-26), pages 1981-1984, XP000929654 ISSN: 0036-8075

### SEQUENCE LISTING

<110> CENTRE NATIONAL DE LA RECHERCHE SCIENTIFIQUE INSTITUT NATIONAL DE LA RECHERCHE AGRONOMIQUE
<120> SOLID SUPPORTS FUNCTIONALISED BY PHOSPHORUS DENDRIMERS, METHODS FOR PREPARING SAME AND USES THEREOF
<130> SGimF644/73PCT
<140> PCT/FR2003/001231 <141> 2003-04-17
<150> FR02/05049
   <151> 2002-04-23
<160> 5
<170> PatentIn version 3.1
<210> 1
   <211> 35
   <212> DNA
   <213> saccharomyces cerevisiae
<400> 1
   gtgatcgttg tatcgaggaa tactccgata ccatt 35
<210> 2
   <211> 15
   <212> DNA
   <213> Artificial Sequence
<220>
   <223> oligonucleotide
<400> 2
   aatggtatcg gagta 15
<210> 3
   <211> 15
   <212> DNA
   <213> Artificial Sequence
<220>
   <223> oligonucleotide
<400> 3
   aatggtaacg gagta 15
<210> 4
   <211> 15
   <212> DNA
   <213> Artificial Sequence
<220>
   <223> oligonuclotide
<400> 4
   aatggtagcg gagta 15
<210> 5
   <211> 15
   <212> DNA
   <213> Artificial sequence
<220>
   <223> Oligonucleotide
<400> 5
   aatggtaccg gagta 15

## Description

La présente Invention est relative à des supports solides fonctionnalisés par des dendrimères phosphorés, à leur procédé de préparation, à leur utilisation pour la préparation de biopuces et aux applications de ces biopuces, en particulier pour l'immobilisation de macromolécules, notamment de macromolécules biologiques telles que des acides nucléiques, des lipides, des protéines (peptides, enzymes, anticorps, ...) ou leurs partenaires moléculaires.

Le développement exponentiel de la génomique et de la pharmacogénomique lié au séquençage non seulement du génome humain, mais également à celui d'animaux, de bactéries, de virus, de plantes, etc... amène les laboratoires de recherche académiques ou industriels à consommer de manière massive des biopuces fiables, faciles à fabriquer.

Or, dans ce cadre particulier, il est primordial de pouvoir disposer de supports solides fonctionnalisés présentant un certain nombre de qualités.

Ces supports doivent en particulier permettre l'immobilisation reproductible de molécules d'intérêt, dans la mesure où une immobilisation reproductible est conditionnelle d'une détection elle-même reproductible.

Ces supports doivent également permettre l'immobilisation des molécules d'intérêt de façon sensible. La sensibilité d'un support solide fonctionnalisé dépend du taux d'immobilisation et de la méthode de détection d'un signal, mais aussi et surtout du niveau du bruit de fond (signal non spécifique). Une diminution du bruit de fond améliore le rapport signal/bruit. En effet, dans un dispositif dans lequel on détecte la présence d'espèces biologiques au voisinage de la surface, le bruit de fond vient essentiellement de l'adsorption non spécifique de molécules y compris de molécules biologiques d'intérêt marquées et qu'il convient par conséquent de limiter. L'idéal est donc d'obtenir un support qui possède un bruit de fond très bas et une intensité de détection du signal élevée.

D'autre part, et notamment dans le cas particulier des réactions d'hybridation mettant en oeuvre des acides nucléiques, l'accrochage des sondes sur la surface d'un support solide doit garantir l'intégrité de la séquence et la stabilité du dépôt. Après l'étape d'hybridation les résultats doivent être reproductibles d'un support à l'autre. A cet effet, les sondes doivent être espacées de la surface du support solide afin de ne pas perturber l'hybridation avec les acides nucléiques cibles. Ceci permet de s'approcher des conditions d'hybridation en solution en mimant une hybridation en 3 dimensions plutôt qu'en 2 dimensions classiquement obtenues par la technique lame de verre. Un espaceur d'au moins une quarantaine d'atomes (4-5 nm) est nécessaire pour éviter les contraintes stériques entre l'ADN et la surface du support.

Par ailleurs, il est important de pouvoir disposer de supports solides fonctionnalisés réutilisables. La possibilité de pouvoir disposer d'un support réutilisable est d'un grand intérêt car elle autorise l'analyse de plusieurs échantillons biologiques avec un seul et même dispositif, ce qui permet d'effectuer des comparaisons quantitatives. De plus, les supports réutilisables autorisent la réalisation de plusieurs mesures sur un même échantillon et permettent ainsi l'amélioration des résultats d'un point de vue statistique.

A l'heure actuelle, différents types de procédés de préparation des biopuces ont déjà été proposés.

Les biopuces, et en particulier les biopuces à acides nucléiques, peuvent être fabriquées soit par synthèse *in situ* soit par immobilisation de sondes.

Dans le premier cas (synthèse *in situ*), les sondes sont des oligonucléotides qui sont synthétisés étape par étape directement sur le support et qui ont une taille généralement comprise entre 20 et 30 bases. Ce procédé de fabrication permet d'avoir accès à des puces à haute densité (McGall et al, J. Am. Chem. Soc., 1997, 119, 5081-5090).

Dans le deuxième cas (immobilisation de sondes), les sondes sont présynthétisées puis immobilisées sur le support. Deux types d'interactions avec la surface peuvent alors entrer en jeu.

L'acide nucléique sonde peut être maintenu sur la surface par des interactions électrostatiques entre les groupements phosphates du squelette chargés négativement et la surface modifiée du support chargée positivement. A titre d'exemple, ce type de biopuce peut être obtenu par recouvrement de la surface avec de la poly-L-lysine ou par silanisation avec un amino-silane (Zammatteo et al, Anal. Biochem., 2000, 280, 143-150 ; Eisen et al, Methods in Enzymol, 1999, 303, 179-205). Dans ce type d'immobilisation, la sonde est généralement couchée sur le support ce qui peut entraîner des problèmes d'accessibilité lors de l'étape d'hybridation. De plus, ce mode d'interactions ioniques n'est pas suffisamment stable pour permettre une réutilisation de la biopuce.

L'acide nucléique sonde peut également être greffé sur le support par le biais d'interactions covalentes. Généralement les liaisons entre les sondes et le support sont établies par une des extrémités 3' ou 5' de l'acide nucléique permettant une accessibilité de la sonde sur toute sa longueur d'où une meilleure qualité de la réponse en terme d'hybridation. Plusieurs combinaisons de fonctionnalisation du support et des acides nucléiques peuvent être rencontrées. Le support peut, par exemple, comporter des fonctions nucléophiles telles que des fonctions -NH₂ ou -SH et l'acide nucléique à greffer comporte alors une fonction électrophile telle que par exemple une fonction -CHO, -NCS, -NHS ou encore -COOR, et inversement. Selon une autre variante, le support et l'acide nucléique peuvent être nucléophiles et un espaceur di-électrophile permettra le couplage entre les deux entités. Enfin le support et la sonde peuvent être électrophiles et le couplage sera réalisé grâce à un espaceur di-nucléophile.

Les biopuces fabriquées actuellement sont, pour une très grande majorité, réalisées en utilisant le verre comme support.

Les plus utilisées possèdent des surfaces fonctionnalisées avec un espaceur terminé par une fonction -NH₂. Leur efficacité en terme d'accrochage et d'hybridation a été confirmée mais aucune réutilisation de ce type de lame n'est possible, les interactions entre les sondes et le support étant de type ionique.

D'autres lames comportant à leur surface des fonctions aldéhyde sont commercialisées. Ces fonctions sont introduites par utilisation d'un silane-aldéhyde, espaceur simple, qui ne présente qu'une seule fonction d'ancrage par molécule que ce soit du côté du support ou du côté des acides nucléiques. Or il a été montré que l'ancrage par plusieurs points sur la surface est important (Zhao et al., Nucl. Acids Res., 2001, 29, 955-959 ; demande internationale WO 01/51689) puisqu'il permet d'assurer une augmentation des sites de fixation des sondes sur le support ce qui entraîne une meilleure réponse en terme d'hybridation.

Une autre façon d'obtenir une meilleure sensibilité de détection est notamment reportée dans les travaux de M. Beier et J.D. Hoheisel, Nucl. Acids Res., 1999, 27, 1970-1977. Les auteurs ont construit, sur une lame de verre, des molécules ramifiées à six branches dans le but d'augmenter la densité de sondes. La nature de ces espaceurs ramifiés permet aussi de moduler la nature hydrophile ou hydrophobe de la surface. Leur procédé de préparation inclut au total huit étapes de synthèse, à partir d'une lame aminée, pour obtenir la biopuce ce qui limite fortement leur développement industriel. De plus, les molécules ramifiées sont générées *in situ* de façon non contrôlable, et leur structure n'est pas définie, ce qui ne permet pas de garantir une homogénéité de la surface de la biopuce ainsi obtenue. Tous les liens étant covalents, les auteurs ont montré, que leurs biopuces pouvaient être réutilisées. Il est à noter que la réutilisation des biopuces est courante dans le cas d'un support en Nylon® et a été plus récemment décrite avec du plastique (voir demande internationale WO 00/55627).

Enfin, des travaux récents (Benters et al, Chembiochem., 2001, 2, 686-694) décrivent l'utilisation de dendrimères aminés "PAMAM® starbust" pour la fabrication de biopuces, en sept étapes de synthèse exigeantes en terme de conditions opératoires, et dont certaines ne peuvent être contrôlées puisqu'elles sont réalisées directement sur le verre, pour l'obtention d'un support qui n'est pas stable dans le temps. En effet, l'utilisation de ces biopuces inclut une étape d'activation préalable de la couche de dendrimères avant de réaliser le couplage avec les oligonucléotides. Cette étape génère une surface réactive qui n'est pas stable dans le temps puisque les auteurs indiquent que les sondes doivent être greffées immédiatement après l'activation. Ceci limite fortement la commercialisation de ce type de support.

Le développement exponentiel de la génomique et de la pharmacogénomique lié au séquençage non seulement du génome humain, mais également à celui d'animaux, de bactéries, de virus, de plantes, etc... amène les laboratoires de recherche académiques ou industriels à consommer de manière massive des biopuces fiables, faciles à fabriquer. La réutilisation est un critère supplémentaire qui permet de valider de manière statistique les essais biologiques. C'est donc afin de remédier à l'ensemble de ces inconvénients et de pourvoir à des biopuces réutilisables pouvant être fabriquées à moindre coût, en peu d'étapes, de manière contrôlée et reproductible et présentant une excellente stabilité et une très bonne sensibilité de détection, que les Inventeurs ont mis au point ce qui fait l'objet de l'Invention.

La présente Invention a donc pour premier objet un support solide caractérisé par le fait qu'il comprend au moins une surface fonctionnalisée de façon covalente par des dendrimères phosphorés possédant un noyau central qui contient au moins deux groupements fonctionnels et comportant à leur périphérie plusieurs fonctions aptes à permettre la fixation desdits dendrimères sur ladite surface ainsi que la fixation ou la synthèse *in situ* de molécules d'intérêt, lesdits dendrimères ayant une taille comprise entre 1 et 20 nm, et par le fait que les dendrimères sont choisis parmi ceux qui sont constitués :
- d'une couche centrale sous la forme d'un noyau central P₀, éventuellement phosphoré, comprenant de 2 à 12 groupements fonctionnalisés,
- de n couches intermédiaires, identiques ou différentes, chacune desdites couches intermédiaires étant constituée d'unités P₁ répondant à la formule (I) 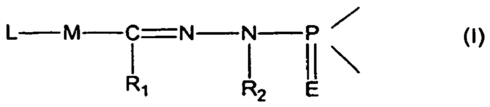 suivante:
   dans laquelle :
   **L** est un atome d'oxygène, de phosphore, de soufre ou d'azote,
   **M** représente l'un des groupes suivants :
      - un groupe aromatique non substitué, di-, tri- ou tétrasubstitué par des groupes alkyles, des groupes alcoxy, des groupements insaturés du type oléfinique en C₁-C₁₂, azoïques, acétylénique, tous ces groupes pouvant incorporer ou non des atomes de phosphore, d'oxygène, d'azote, de soufre ou des halogènes, ou
      - un groupe alkyle ou alcoxy comportant plusieurs substituants tels que définis lorsque M est un groupe aromatique,
   **R₁ et R₂**, qui peuvent être identiques ou différents, représentent un atome d'hydrogène ou l'un des groupes suivants : alkyle, alcoxy, aryle, comportant ou non des atomes de phosphore, d'oxygène, de soufre, d'azote ou des halogènes avec R₂ étant le plus souvent différent de R₁,
   **n** est un nombre entier compris entre 1 et 11,
   **E** est un atome d'oxygène, de soufre ou d'azote, ledit atome d'azote pouvant être lié à un groupe alkyle, alcoxy ou aryle, tous ces groupes pouvant incorporer ou non des atomes de phosphore, d'oxygène, d'azote, de soufre ou des halogènes,
- une couche externe constituée d'unités P₂, identiques ou différentes, et répondant à la formule II suivante : 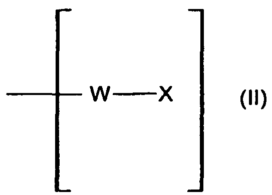 dans laquelle :
   **W** représente l'un des groupes suivants : alkyle, alcoxy, aryle, tous ces groupes comportant ou non des atomes de phosphore, d'oxygène, d'azote, de soufre ou des halogènes,
   **X** représente un groupement aldéhyde, thiol, amine, époxyde, acide carboxylique, alcool ou phénol.

Dans la suite de la description, de tels supports solides fonctionnalisés par des dendrimères phosphorés sont appelés « dendrilames ».

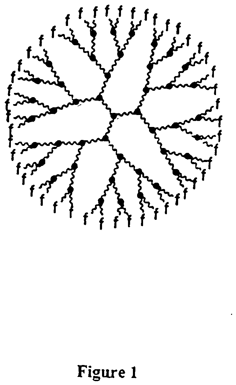

Les dendrimères sont des polymères, isomoléculaires à structure ramifiée et définie, formés d'un noyau central (coeur) à partir duquel sont créées ou greffées des ramifications. Ces ramifications sont multifonctionnelles, c'est-à-dire qu'elles portent, en particulier en périphérie, plusieurs groupements chimiques fonctionnels, identiques ou différents, choisis selon les propriétés que l'on souhaite conférer au dendrimère. Chaque nouvelle génération est obtenue par introduction d'un nouveau niveau de ramifications. L'ensemble des points de jonctions des branches situés à une égale distance du coeur correspond à une génération. Une représentation schématique d'un dendrimère de génération 4, obtenu à partir d'un coeur trifonctionnel, est donnée sur la figure 1 annexée.

De façon préférentielle, le noyau central P₀ de ces dendrimères est sélectionné dans le groupe constitué par le groupement de formule générale IIIa : 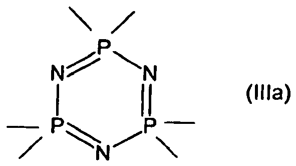 et le groupement de formule générale IIIb : 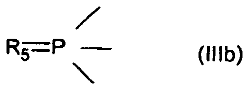 dans lequel R₅ représente un atome de soufre, d'oxygène ou d'azote.

Selon une forme de réalisation avantageuse de l'Invention, les dendrimères sont choisis parmi les composés dans lesquels le groupe de formule (I) ci-dessus représente le groupe suivant : 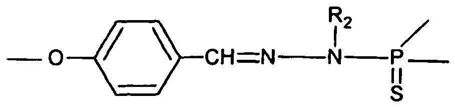 dans lequel R₂ représente un radical alkyle en C₁-C₁₂ et plus particulièrement un radical méthyle ;
et le groupe de formule (II) représente l'un des deux groupes suivants : 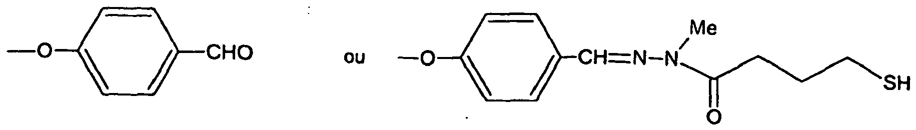 et dans lesquels le nombre de générations varie de préférence entre 1 et 6.

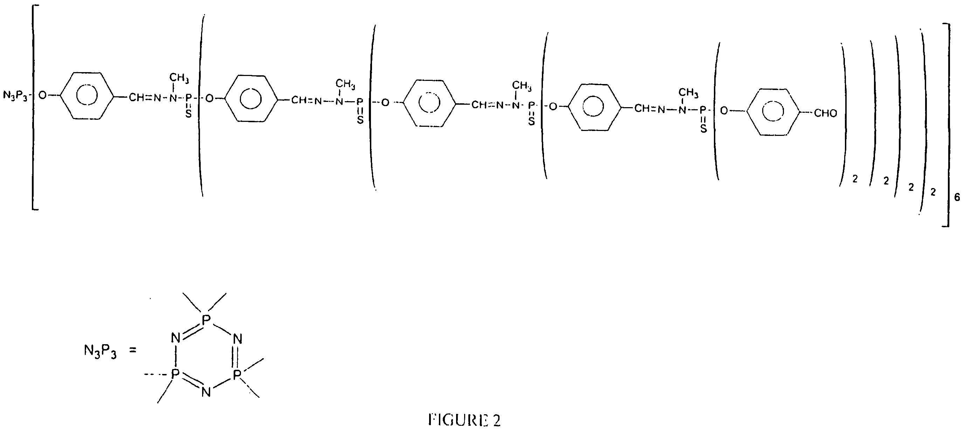

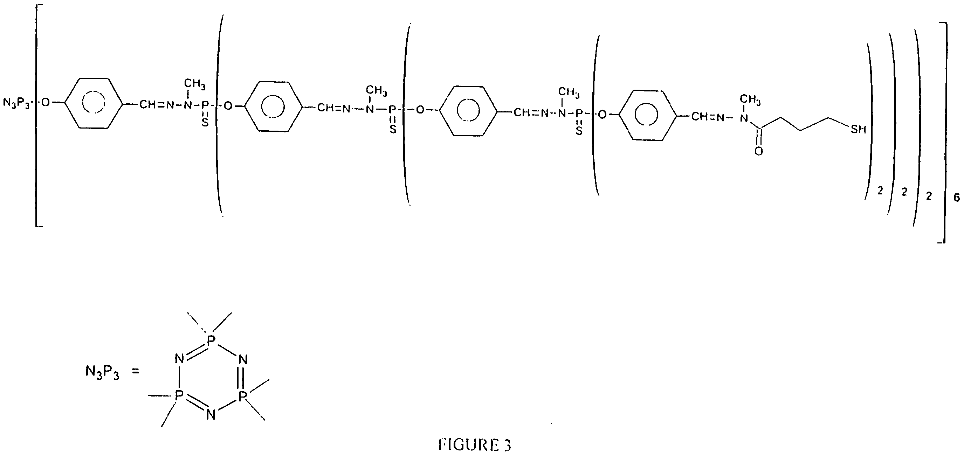

Des exemples de tels dendrimères sont donnés sur les figures 2 et 3 annexées qui représentent respectivement la structure d'un dendrimère de génération 4 à terminaisons aldéhyde et la structure d'un dendrimère de génération 3 à terminaisons thiol.

Parmi les supports solides pouvant être fonctionnalisés conformément à l'Invention, on peut en particulier citer les supports comportant au moins une surface siliciée telle que les lames, les billes et les capillaires en verre, les supports en silicium, en plastique et les supports métalliques tels que par exemple les plots d'or.

Les dendrimères utilisés conformément à l'Invention peuvent être synthétisés de façon connue de l'Homme du métier, par répétition d'une même séquence réactionnelle permettant l'obtention d'une nouvelle génération à la fin de chaque cycle de synthèse et par conséquent d'un nombre croissant de branches et de fonctions périphériques toutes identiques (Tomalia D.A., Ang. Chem. Int. Ed., 1990, 29, 138 ; Launay et al, Angew. Chem. Int. Ed. Engl., 1994, 33, 1589 et J. Organomet. Chem., 1997, 529, 51).

En particulier, ces dendrimères peuvent être construits étape par étape à partir d'un noyau central possédant au moins deux groupements fonctionnels tel que par exemple l'hexachlorocyclotriphosphazène : N₃P₃Cl₆, qui possède six groupements fonctionnels.

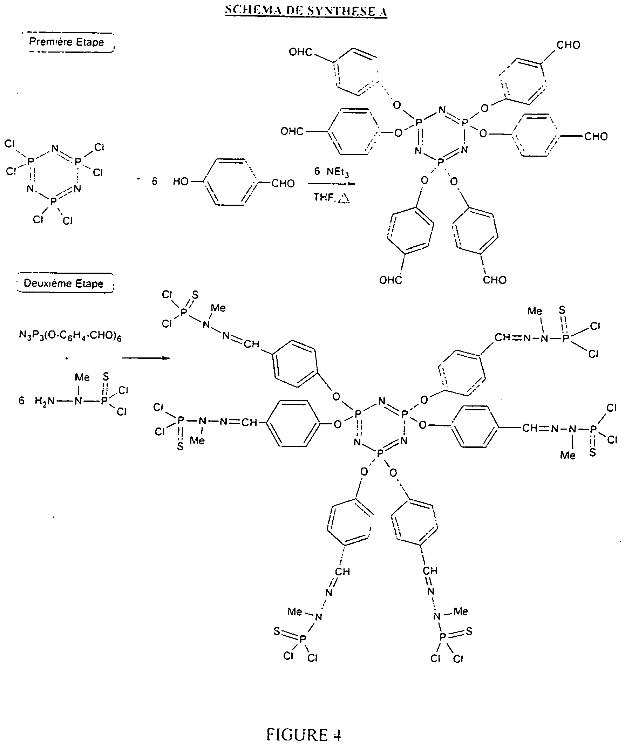

De façon plus précise, ces dendrimères sont généralement construits par la répétition de deux étapes à partir du coeur. La première étape est la substitution des atomes de chlore en milieu basique par un composé difonctionnel ayant une fonction alcool et une fonction aldéhyde, par exemple le 4-hydroxybenzaldéhyde. La deuxième étape crée les points de ramification et consiste en une réaction de condensation avec un composé ayant deux types de fonctions : un groupement NH₂ et au moins un groupement PCl₂ comme par exemple le composé H₂NNMeP(S)Cl₂. Les deux premières étapes de la méthode de synthèse à partir du coeur hexafonctionnel sont représentées sur le schéma de synthèse A représenté sur la figure 4 annexée.

Une suite itérative des étapes de synthèse permet de construire des dendrimères de génération croissante.

Le tableau I ci-après indique le nombre de fonctions terminales présentes à la périphérie des dendrimères de différentes générations obtenus à partir du coeur N₃P₃ ainsi que leur taille en Angstrôms :

**Tableau I**

| **Génération** | **Nombre de fonctions** | **Taille (A)** |
|---|---|---|
| 1 | 12 | 30 |
| 2 | 24 | 45 |
| 3 | 48 | 60 |
| 4 | 96 | 75 |
| 5 | 192 | 90 |
| 6 | 384 | 105 |
| 7 | 768 | 120 |

La présente Invention a également pour objet un procédé de préparation d'un support solide conforme à l'Invention (dendrilame), caractérisé par le fait qu'il comprend une étape de formation d'une liaison covalente entre des dendrimères phosphorés possédant un noyau central qui contient au moins deux groupements fonctionnels, lesdits dendrimères comportant à leur périphérie plusieurs fonctions aptes à permettre leur fixation sur ladite surface et la fixation ou la synthèse *in situ* de molécules d'intérêt, et la surface fonctionnalisée ou non d'un support solide pour obtenir un support solide fonctionnalisé de façon covalente par lesdits dendrimères.

Selon une première forme de réalisation du procédé conforme à l'Invention, les dendrimères comportent à leur périphérie des fonctions permettant l'accrochage direct par l'intermédiaire d'une liaison covalente de ces derniers sur la surface non préalablement fonctionnalisée dudit support solide. C'est le cas, en particulier, lorsque les dendrimères comportent à leur périphérie des fonctions thiol et que le support solide comprend une surface d'or.

Selon une seconde forme de réalisation du procédé conforme à l'Invention, et lorsque la surface du support solide utilisé ne comprend pas de fonctions compatibles avec les fonctions périphériques du dendrimères utilisé, il est alors nécessaire de fonctionnaliser préalablement ladite surface au moyen de fonctions aptes à permettre la fixation covalente desdits dendrimères.

Selon cette seconde variante, le procédé conforme à l'Invention est alors caractérisé par le fait qu'il comprend les étapes suivantes :
a) la fonctionnalisation d'au moins une surface d'un support solide par des fonctions aptes à permettre la fixation de dendrimères phosphorés possédant un noyau central qui contient au moins deux groupements fonctionnels, lesdits dendrimères comportant à leur périphérie plusieurs fonctions aptes à permettre leur fixation sur ladite surface ainsi fonctionnalisée et la fixation ou la synthèse *in situ* de molécules d'intérêt ;
b) la préactivation éventuelle des fonctions du support pour obtenir une surface fonctionnalisée activée,
c) la formation d'une liaison covalente entre lesdits dendrimères et ladite surface fonctionnalisée et éventuellement activée, pour obtenir un support solide fonctionnalisé de façon covalente par lesdits dendrimères.

Selon une forme de réalisation avantageuse de l'Invention, l'étape a) de fonctionnalisation de la surface du support solide est réalisée par silanisation au moyen d'un réactif de silanisation comportant des fonctions aptes à fixer les dendrimères, telles que par exemple des groupements amine.

A titre d'exemple, l'étape de silanisation peut être réalisée en utilisant un réactif de silanisation aminé tel que par exemple le 3-aminopropyltriéthoxysilane (Sigma), l'aminopropyldiéthoxyméthylsilane ou bien encore l'aminopropylmonoéthoxydiméthylsilane.

Selon une forme de réalisation préférée de l'Invention, l'étape de fixation covalente directe des dendrimères (cas de la première variante) et l'étape a) de silanisation de la surface du support (cas de la deuxième variante) sont précédées d'une étape de nettoyage de la surface du support en milieu basique, acide et/ou oxydant.

Dans le cas particulier de la deuxième variante du procédé conforme à l'Invention, ces deux étapes de lavage et de silanisation sont résumées sur le schéma de synthèse B ci-dessous :

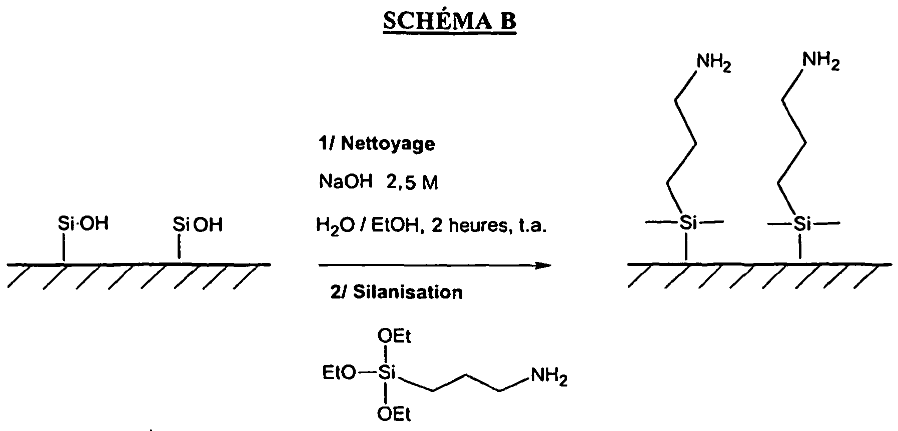

L'étape b) optionnelle de préactivation sert à réactiver si nécessaire les fonctions amine fixées sur le support à l'issue de l'étape de silanisation. En effet, le stockage, même court, des supports solides aminés peut entraîner une protonation des groupements NH₂. Il est donc souvent préférable de réactiver les fonctions amine en milieu basique avant de faire réagir les dendrimères. Dans ce cas particulier, les étapes b) et c) sont représentés sur le Schéma C ci-après :

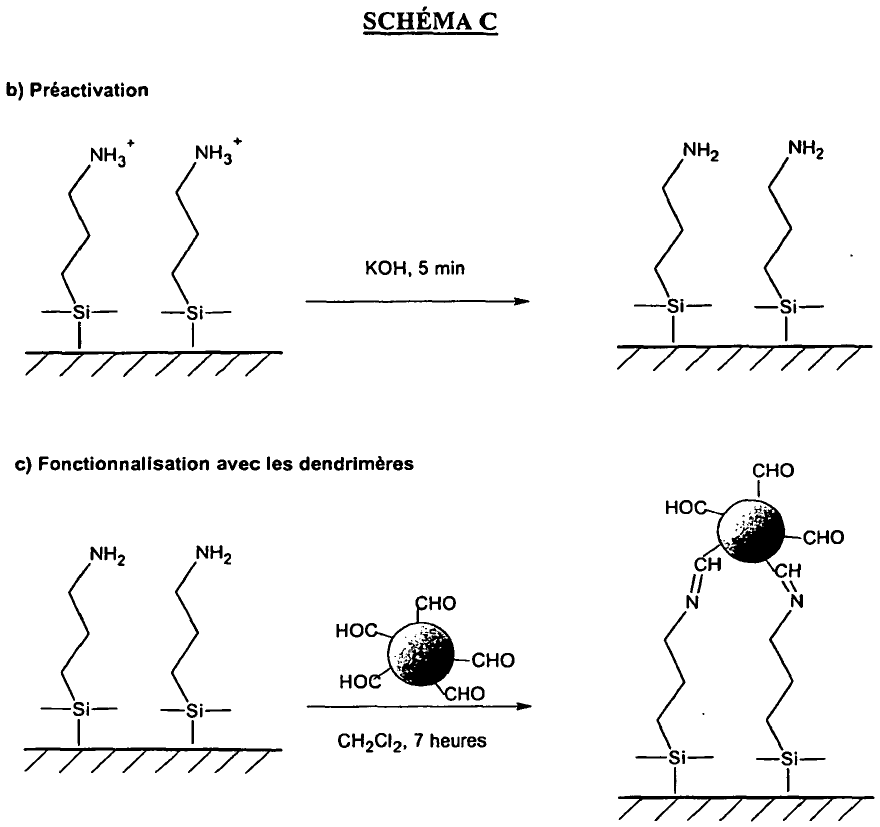

Selon une forme de réalisation avantageuse du procédé conforme à l'Invention, cette étape de préactivation est réalisée par traitement du support à l'aide d'un agent alcalinisant tel que par exemple la potasse, pendant une durée comprise entre 2 et 20 minutes environ.

Selon une forme de réalisation avantageuse de l'Invention, l'étape de fixation covalente des dendrimères, consiste à :
- préparer une solution desdits dendrimères dans un solvant, tel que par exemple le dichlorométhane ou le tetrahydrofurane,
- mettre ladite solution de dendrimères en contact avec la surface éventuellement fonctionnalisée et éventuellement activée, pendant une durée comprise entre 10 minutes et 24 heures environ, de préférence entre 2 et 8 heures environ, à une température de préférence comprise entre 4 et 50°C environ.

A l'issue de l'étape de fixation covalente des dendrimères, les supports conformes à l'Invention (dendrilames) sont de préférence rincés puis séchés.

L'étape de rinçage est de préférence réalisée à l'aide d'un solvant organique tel que le dichlorométhane ou le tetrahydrofurane puis à l'aide d'un alcool inférieur tel que l'éthanol.

Le séchage des dendrilames peut par exemple être effectué à l'air comprimé, sous courant d'azote ou bien encore par centrifugation.

Les dendrilames ainsi obtenues peuvent ensuite être stockées et/ou directement utilisées pour l'immobilisation et/ou la synthèse *in situ* de molécules, en particulier de molécules biologiques.

Les dendrilames conformes à l'Invention peuvent en particulier être stockées pendant au moins deux mois, sans qu'aucune altération des fonctions situées à la périphérie des dendrimères ne se produise.

La présente Invention a donc également pour objet l'utilisation d'un support solide fonctionnalisé par des dendrimères phosphorés conformes à l'Invention (dendrilames), comme support pour l'immobilisation et/ou la synthèse *in situ* de molécules d'intérêt telles que par exemple des molécules d'acide nucléique (oligonucléotides, produits PCR...), des lipides, des protéines (peptides, enzymes, anticorps, ...) ou leurs partenaires moléculaires.

L'Invention a aussi pour objet une biopuce caractérisée par le fait qu'elle est constituée d'un support solide comportant au moins une surface fonctionnalisée par des dendrimères phosphorés sur lesquels sont fixées de façon covalente des molécules d'intérêt telles que des acides nucléiques (oligonucléotides, produits PCR...), des lipides, des protéines (peptides, enzymes, anticorps, ...) ou leurs partenaires moléculaires.

Selon l'Invention, de telles biopuces sont appelées « dendripuces ».

De telles dendripuces possèdent un bruit de fond très bas et une intensité de signal élevée par exemple après hybridation d'acides nucléiques avec des cibles fluorescentes. Ces "dendripuces" présentent l'avantage d'être réutilisables sans perte significative du signal mais surtout sans augmentation du bruit de fond. Ceci entraîne une réduction significative des coûts d'analyse et permet d'obtenir des données statistiques fiables pour une analyse donnée. Ces dendripuces sont de plus obtenues en peu d'étapes de synthèse (trois à cinq) ; celles-ci se déroulant dans des conditions opératoires non contraignantes, de manière contrôlée et reproductible.

Par ailleurs, du fait de la structure même des dendrimères, ces dendripuces présentent l'avantage de posséder de multiples points d'ancrage sur la surface d'une part et sur les molécules d'intérêt d'autre part, le tout garantissant une excellente stabilité de l'ensemble support-dendrimères-molécules d'intérêt.

Un autre objet de l'Invention est un procédé de préparation d'une dendripuce telle que définie ci-dessus, caractérisé par le fait qu'il consiste à mettre en contact un support solide ayant au moins une surface fonctionnalisée par des dendrimères phosphorés comportant à leur périphérie des fonctions aptes à permettre la fixation covalente de molécules d'intérêt avec une solution tampon renfermant des molécules d'intérêt préalablement fonctionnalisées par, ou comportant déjà, un ou plusieurs groupements aptes à former une liaison covalente avec lesdites fonctions périphériques des dendrimères.

Selon une première forme de réalisation de ce procédé, et lorsque les fonctions périphériques des dendrimères utilisés sont des fonctions aldéhydes, alors les molécules d'intérêt sont de préférence préalablement fonctionnalisées par, ou contiennent déjà, une ou plusieurs fonctions amine, ou sont préalablement fonctionnalisées par une ou plusieurs fonctions oxyamine (-ONH₂) ou hydrazine (-NH-NH₂), et de manière plus générale par toute fonction susceptible de réagir avec une fonction aldéhyde.

Aussi, dans ce cas, les molécules d'intérêt (acides nucléiques aminés, oligonucléotides de tailles variables ou produits PCR), sont déposés sur les "dendrilames" en utilisant, par exemple, le tampon phosphate 0,3 M, pH 9. Lorsque les molécules d'intérêt sont préalablement fonctionnalisées par, ou contiennent déjà, une ou plusieurs fonctions amine, cette étape est suivie d'une réduction des fonctions imines présentes entre les acides nucléiques et les dendrimères d'une part et entre les dendrimères et le support d'autre part (voir Schéma D ci-après). Cette étape de réduction permet également de réduire les fonctions aldéhydes résiduelles en alcool rendant la surface hydrophile. De plus, les alcools ne peuvent pas entraîner de réaction non spécifique avec les cibles marquées (pas d'augmentation du bruit de fond).

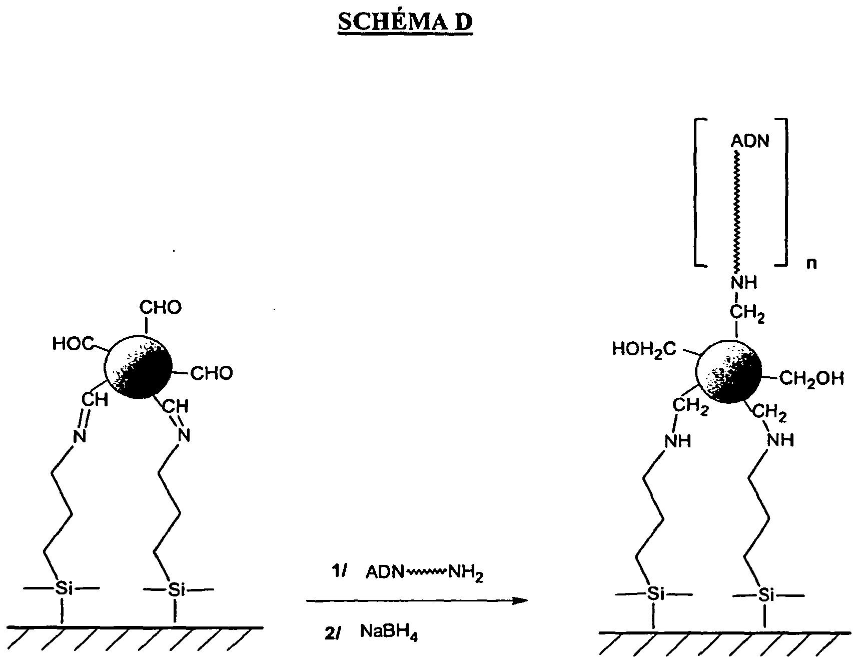

Selon une deuxième forme de réalisation de ce procédé, et lorsque les fonctions périphériques des dendrimères utilisés sont des fonctions thiol, alors les molécules d'intérêt sont de préférence préalablement fonctionnalisées par, ou contiennent déjà, une ou plusieurs fonctions thiol (pour permettre la création d'un pont disulfure), ou sont préalablement fonctionnalisées par une ou plusieurs fonctions iodoacétamido (-NHCO-CH₂-I), et de manière plus générale, par toute fonction susceptible de réagir avec une fonction thiol. Ce type de fonctionnalisation est particulièrement adapté aux supports solides comportant une surface de type silicium ou en or. Dans le cas d'un lien disulfure établi entre le support et les sondes nucléiques, la surface peut être régénérée par une étape de réduction. La surface thiol ainsi obtenue peut être à nouveau "rechargée" avec d'autres séquences oligonucléotidiques ou avec des protéines.

Selon une troisième forme de réalisation de ce procédé, et lorsque les fonctions périphériques des dendrimères utilisés sont des fonctions amines, alors les molécules d'intérêt sont préalablement fonctionnalisées par, ou contiennent déjà une ou plusieurs fonctions aldéhyde, α-oxoaldéhyde, -COOR, -NCS, -NHS, et de manière plus générale, par toute fonction capable de réagir avec une fonction amine.

Selon une quatrième forme de réalisation de ce procédé, et lorsque les fonctions périphériques des dendrimères utilisés sont des fonctions époxyde, alors les molécules d'intérêt sont préalablement fonctionnalisées par, ou contiennent déjà une ou plusieurs fonctions amine, et de manière plus générale, par toute fonction capable de réagir avec une fonction époxyde.

Selon le procédé de préparation des dendripuces conforme à l'Invention, la réaction de fixation covalente des molécules d'intérêt sur les dendrilames est de préférence réalisée à une température comprise entre 4 et 50°C environ, pendant une durée comprise entre 2 et 24 heures environ.

Les dendripuces ainsi obtenues peuvent avantageusement être utilisées en tant qu'outil de diagnostic miniaturisé, en fonction de la nature des molécules d'intérêt fixées, comme puces à ADN par exemple pour réaliser des réactions d'hybridation avec des cibles complémentaires, comme puces à peptides, à polypeptides ou à protéines par exemple pour la détection de réponses de type antigène-anticorps par l'utilisation de réactifs marqués, fluorescents, radioactifs ou marqués chimiquement.

Les dendripuces conformes à la présente Invention peuvent également être utilisées comme puces à polypeptides pour le criblage de molécules et pour l'analyse des relations entre molécules, de type ligand-récepteur.

De telles dendripuces présentent en particulier l'avantage d'être réutilisables.

Outre les dispositions qui précèdent, l'Invention comprend encore d'autres dispositions qui ressortiront de la description qui va suivre, qui se réfère à deux exemples de préparation de dendrilames en verre fonctionnalisées par des dendrimères de différentes générations et à extrémités aldéhyde, à un exemple de préparation de biopuces à partir de ces dendrilames, à un exemple d'immobilisation de molécules d'acide nucléique sur des biopuces conformes à l'Invention, à un exemple de synthèse d'un dendrimère de génération 3 et à extrémités thiol et à un exemple de préparation de dendrilames avec des dendrimères de génération 3 à extrémités thiol, ainsi qu'aux figures 1 à 7 dans lesquelles :
- la figure 1 représente une vue schématique d'un dendrimère de génération 4, obtenu à partir d'un coeur trifonctionnel ;
- la figure 2 représente la formule d'un dendrimère phosphoré de génération 4 comportant des fonctions périphériques terminales aldéhydes ;
- la figure 3 représente la formule d'un dendrimère phosphoré de génération 3 à fonctions périphériques terminales thiol ;
- la figure 4 est un schéma de synthèse A représentant les deux premières étapes du procédé de préparation d'un dendrimère à coeur phosphoré ;
- la figure 5 représente les images des signaux de fluorescence obtenus après 10 cycles d'hybridation/déshybridation sur des dendripuces conformes à l'Invention ;
- la figure 6 représente les images des signaux de fluorescence obtenus après 10 cycles d'hybridation/déshybridation sur des dendripuces conformes à l'Invention en fonction de la concentration en oligonucléotides cibles ;
- la figure 7 représente les images des signaux de fluorescence obtenus lors d'une étude concernant la recherche de mutations sur des oligonucléotides.

### EXEMPLE 1 : PRÉPARATION D'UN SUPPORT SOLIDE CONSTITUE PAR UNE LAME DE VERRE FONCTIONNALISÉE PAR DES DENDRIMÈRES PHOSPHORES A EXTRÉMITÉS ALDÉHYDE ("DENDRILAMES")

### 1) Première étape : Préactivation des lames de verre

Des lames de verre commerciales, fonctionnalisées par des groupements amine (CORNING®-CMT-GAPS Amino-Silane Coated Slides) sont immergées dans une solution de aqueuse de potasse (8 %) pendant 20 minutes sous agitation à l'aide d'un agitateur orbital à une vitesse de 30 rotations par minute (Heidolph Instruments Polymax 1040). Les lames sont ensuite abondamment rincées avec de l'eau milliQ (3 lavages de 5 minutes chacun) et sont enfin séchées sous courant d'azote.

### 2) Deuxième étape : Préparation des "dendrilames"

Dans cet exemple, trois types de dendrimères différents ont été utilisés :
- Dendrimères G3 : Dendrimères de troisième génération comportant un coeur phosphoré de formule (IIIa) telle que décrite ci-dessus et comportant à leur périphérie 48 fonctions aldéhydes ;
- Dendrimères G4: Dendrimères de quatrième génération comportant un coeur phosphoré de formule (IIIa) telle que décrite ci-dessus et comportant à leur périphérie 96 fonctions aldéhydes ;
- Dendrimères G5 : Dendrimères de cinquième génération comportant un coeur phosphoré de formule (IIIa) telle que décrite ci-dessus et comportant à leur périphérie 192 fonctions aldéhydes ;

Les lames préactivées sont immergées dans une solution de dendrimères G3, G4 ou G5 à 0,1% dans du dichlorométhane. Elles sont agitées pendant 6 heures à température ambiante (30 rotations/mn). Elles sont ensuite rincées avec du dichlorométhane (2 lavages de 5 minutes chacun) puis avec de l'éthanol (1 lavage de 5 minutes) et sont enfin séchées sous courant d'azote.

On obtient ainsi des lames de verre dont la surface est fonctionnalisée par des dendrimères de G3, G4 ou G5 (dendrilames : DL-A-G3 ; DL-A-G4 et DL-A-G5). Ces dendrilames peuvent ensuite être utilisées pour l'immobilisation de molécules d'intérêt.

### EXEMPLE 2 : PRÉPARATION D'UN SUPPORT SOLIDE CONSTITUÉ PAR UNE LAME DE VERRE FONCTIONNALISÉE PAR DES DENDRIMÈRES PHOSPHORÉS A EXTRÉMITÉS ALDÉHYDE ("DENDRILAMES")

### 1) Première étape : Nettoyage des lames de verre

Des lames de verre commerciales (Gold-Seal-Microslides®, Polylabo) sont placées sur un portoir et celui-ci est immergé dans une solution alcaline de lavage constituée de 50 g de soude dans 200 ml d'eau milliQ et de 300 ml d'éthanol à 95%. Les lames sont agitées pendant 2 heures à température ambiante. Elles sont ensuite abondamment rincées avec de l'eau milliQ (3 lavages de 5 minutes chacun) et séchées sous courant d'azote ou par centrifugation.

### 2) Deuxième étape : Silanisation

Les lames de verre ainsi nettoyées sont immergées dans une solution de 3-aminopropyltriéthoxysilane (GAPS, Aldrich) à 10 % dans l'éthanol à 95 % et sont placées sous agitation pendant une nuit à température ambiante. Elles sont ensuite laissées pendant 30 minutes à l'air libre, rincées avec de l'éthanol à 95 % (2 lavages de 5 minutes chacun) puis avec de l'eau milliQ (1 lavage de 5 minutes et 1 lavage de 2 minutes avec sonication) avant d'être séchées sous courant d'azote. Elles sont ensuite placées pendant 3 heures à une température de 120°C.

### 3) Troisième étape : Préactivation

Les lames silanisées sont placées dans une solution aqueuse de potasse (8 %) pendant 5 minutes, sous agitation à température ambiante. Elles sont ensuite rincées avec de l'eau milliQ (3 lavages de 5 minutes chacun) avant d'être séchées sous courant d'azote.

### 4) Ouatrième étape : Fonctionnalisation avec les dendrimères

Les lames préactivées sont placées dans une solution de dendrimères G4 tel que décrit ci-dessus à l'exemple 1 (0,1 % dans du dichlorométhane) pendant 7 heures sous agitation à température ambiante. Elles sont ensuite lavées avec du dichlorométhane (2 lavages de 5 minutes chacun) puis avec de l'éthanol (1 lavage de 5 minutes) avant d'être séchées sous courant d'azote.

On obtient ainsi un support solide fonctionnalisé par des dendrimères phosphorés à fonctions aldéhydes (dendrilame : DL-B-G4). Cette dendrilame peut ensuite être utilisée pour l'immobilisation de molécules d'intérêt.

### EXEMPLE 3 PRÉPARATION DE BIOPUCES A PARTIR DES DENDRILAMES PRÉPARÉES AUX EXEMPLES 1 ET 2 : "DENDRIPUCES"

### 1) Dépôt des oligonucléotides

### a) Oligonucléotides utilisés

Oligonucléotide ON1 : 35 mer-amine ayant la séquence SEQ ID N°1 suivante :
⁵' -GTG-ATC-GTT-GTA-TCG-AGG-AAT-ACT-CCG-ATA-CCATT -^{3'}, modifié en position 5' par un groupement - NH₂-(CH₂)₆-·

Cet oligonucléotide, qui est modifié à son extrémité 5' par un bras à 6 carbones terminé par une fonction amine (Eurogentec, Belgique), permet la réaction avec les fonctions aldéhydes présentes sur la dendrilame telle que décrite ci-dessus aux exemples 1 et 2.

Oligonucléotide ON2 : 35 mer-oxyamine ayant la séquence SEQ ID N°1 décrite ci-dessus et modifié en position 5' par un - H₂NO-(CH₂)₆-.

La séquence SEQ ID N°1 des oligonucléotides ON1 et ON2 correspond à une partie du gène GPH1 de la levure *Saccharomyces cerevisiae.*

Les dépôts des oligonucléotides ont été effectués en utilisant le robot Eurogridder® (Eurogentec, Seraing, Belgique) et ont été espacés de 200 µm.

### b) Dépôts sur les dendrilames

On utilise les dendrilames DL-A-G3, DL-A-G4, DL-A-G5 et DL-B-G4 telles que préparées ci-dessus aux exemples 1 et 2.

Sur les dendrilames DL-A-G3, DL-A-G4, DL-A-G5 préparées à l'exemple 1, les oligonucléotides ON1 sont déposés dans un tampon phosphate 0,3 M (pH 9) contenant des quantités croissantes de diméthylsulfoxide (DMSO) (0 ; 10 ; 20 ; 30 ; 40 ou 50 %, v/v) en utilisant le robot Eurogridder® (Eurogentec, Belgique). Les volumes déposés sont de 2 nl et les concentrations varient de 1µM à 10 µm (1 ; 2 ; 5 et 10 µM). Après dépôt les lames sont laissées pendant une nuit à température ambiante. Elles sont ensuite immergées dans une solution aqueuse de borohydrure de sodium (NaBH₄, 3,5 mg/mL) (étape de réduction des fonctions imines) et sont agitées pendant 3 heures à température ambiante. Elles sont rincées avec de l'eau MilliQ (3 lavages de 5 minutes chacun) et sont séchées sous courant d'azote ou par centrifugation. On obtient les dendripuces (DP) suivantes DP1-A-G3 ; DP1-A-G4 et DP1-A-G5.

Sur les dendrilames DL-B-G4 préparées à l'exemple 2, les oligonucléotides ON1 sont déposés dans le tampon phosphate 0,3 M (pH 9) en utilisant le robot de dépôt. Les volumes déposés sont de 2 nl et la concentration est de 10 µM. Après dépôt les lames sont laissées pendant une nuit à une température ambiante. Elles sont ensuite immergées dans une solution aqueuse de borohydrure de sodium (NaBH₄, 3,5 mg/mL) (étape de réduction des fonctions imines) et sont agitées pendant 3 heures à température ambiante. Elles sont rincées avec de l'eau MilliQ (3 lavages de 5 minutes chacun) et sont séchées sous courant d'azote ou par centrifugation. On obtient des dendripuces DP1-B-G4.

Les oligonucléotides ON2 ont également été déposés sur les dendrilames préparées à l'exemple 2.

Pour ce faire, les oligonucléotides ON2 sont déposés dans l'eau MilliQ. Les volumes déposés sont de 0,2 µl et les concentrations varient de 1 µM à 10 µM (1 µM, 5 µM et 10 µM). Sur la même lame les oligonucléotides ON1 ont été déposés dans le tampon phosphate 0,3 M dans les mêmes conditions de volume et de concentration. Les lames sont laissées pendant 7 heures à température ambiante. Elles sont ensuite immergées dans une solution aqueuse de borohydrure de sodium (NaBH₄, 3,5 mg/mL) et sont agitées pendant 3 heures à température ambiante. Elles sont ensuite rincées avec de l'eau MilliQ (1 lavage de 5 minutes), avec une solution aqueuse de dodécylsulfate de sodium (SDS) à 0,2 % (1 lavage de 5 minutes) et à nouveau avec de l'eau (5minutes). Elles sont enfin séchées sous courant d'azote. On obtient des dendripuces DP2-B-G4.

On obtient ainsi différentes sortes de biopuces correspondant à des dendrilames sur lesquelles ont été fixés des oligonucléotides ("dendripuces"). Ces dendripuces peuvent ensuite être utilisées dans des réactions d'hybridation.

### EXEMPLE 4 : HYBRIDATION

### 1) Matériel et méthodes

### 1-a) Tampons utilisés

Les différents tampons utilisés lors des étapes d'hybridation sont reportés ci-dessous ; ils ont été préparés à partir des différentes solutions commerciales suivantes :
- 20 x SSC (citrate de sodium 0,3 M ; NaCl 3 M, pH environ 7 ; Sigma)
- 20 x SSPE (Tampon Phosphate 0,2 M, 2,98 M NaCl, 0,02 M EDTA, pH environ 7,4 ; Sigma),
- 50 x Solution de Denhardt (Sigma),
- ADN saumon (9,9 mg/mL, Sigma).
Tampon d'hybridation : SSPE 2 x, SDS 0,1% [ON cible] = 200 nM, pH 7,4 Avec comme oligonucléotides cibles :
ON-Cy5 : ⁵' - Cy5 - AAT GGT ATC GGA GTA - ^{3'}
ON-Cy3 : ^{5'} - Cy3 - AAT GGT ATC GGA GTA - ^{3'}

La séquence de ces oligonucléotides cibles correspond à la séquence SEO ID N°2.
Tampon de lavage : SSPE 2x, SDS 0,1%, pH 7,4 :
Tampon de déshybridation : 2,5 mM Na₂HPO₄, 0,1% SDS

### 1-b) Etape d'hybridation

Les hybridations sont réalisées en ajoutant le volume nécessaire de cibles marquées (de 2 µl à 40 µl suivant la surface à couvrir) sur la zone de dépôt de chaque dendripuce.

Différentes concentrations de cibles à détecter : 200 nM, 100 nM, 20 nM, 10 nM, 2 nM et 1 nM ont été préparées et utilisées au cours des étapes d'hybridation afin d'évaluer la sensibilité de détection des dendripuces conformes à l'Invention.

La goutte est alors recouverte d'une lamelle et l'ensemble est placé dans une chambre d'hybridation (« CMT-Hybridization Chamber » vendue par la société Coming). Les lames sont laissées pendant 15 minutes à température ambiante et sont ensuite lavées en utilisant le tampon de lavage pendant 5 minutes à température ambiante. Elles sont enfin séchées sous courant d'azote.

### 1-c) Lecture des lames

La lecture des lames est réalisée en utilisant un scanner Axon équipé de deux lasers permettant une lecture à des longueurs d'ondes d'excitation de 532 nm et de 635 nm (Cy3 et Cy5 respectivement). La fluorescence émise par les fluorochromes après excitation est détectée par un tube photo-multiplicateur (PMT). Classiquement le PMT a une valeur comprise entre 450 et 600. Le résultat est obtenu sous forme d'un fichier image avec une résolution de 10 µm/pixel. L'analyse informatique des fichiers images et la quantification de l'intensité de fluorescence ont été réalisées en utilisant le logiciel Genepix®.

### 1-d) Etape de déshybridation

Lorsque la lecture des lames est terminée, on procède à la déshybridation des lames.

Pour ce faire, les dendripuces sont placées dans un tube Flacon de 50 ml rempli avec le tampon de déshybridation décrit ci-dessus. L'ensemble est placé pendant 5 minutes à 95°C. La lame est alors rincée trois fois avec de l'eau milliQ et séchée par centrifugation ou sous courant d'azote. La lame est ensuite scannée afin de s'assurer que la déshybridation a bien eu lieu. Elle est ainsi prête pour être à nouveau utilisée pour une autre hybridation.

### 1-e) Etude de mutations

### Dépôts :

L'oligonucléotide ON1 tel que décrit ci-dessus (10 µM, 2 nl) est déposé sur les dendrilames DL-A-G3, DL-A-G4 ou DL-A-G5 préparées selon l'exemple 1. Les dépôts sont effectués en quatre endroits différents sur la même dendrilame. Les hybridations sont effectuées en utilisant quatre oligonucléotides de 15 bases fonctionnalisés à leurs extrémités 5' par un fluorophore Cy5 et qui contiennent en milieu de séquence les quatre bases possibles T, A, G ou C (Eurogentec). Les séquences de ces oligonucléotides sont les suivantes :
-"15mer Cy5 **T**" (complémentaire) : ^{5'}-Cy5-AAT GGT A**T**C GGA GTA^{3'} (SEQ ID N°2)
- "15mer Cy5 A" : ^{5'} - Cy5-AAT GGT A**A**C GGA GTA ^{3'} (SEQ ID N°3)
- "15mer Cy5 G" : ^{5'} - Cy5-AAT GGT A**G**C GGA GTA ^{3'} (SEQ ID N°4)
- "15mer Cy5 C" : ^{5'} - Cy5-AAT GGT A**C**C GGA GTA ^{3'} (SEQ ID N°5)

### Hybridations

- Tampon d'hybridation : SSC 6x, SDS 0,2%, solution de Denhartd 5x, ADN saumon 10 µg, pH 7 contenant l'oligonucléotide 15mer Cy5 T, A, G ou C à une concentration de 200 nM.
- Tampon de lavage : lavage 1 : SSC 2x, 0,1 % SDS, pH 7 lavage 2 : SSC 0,2x.

On dépose 5 µl de chacune des solutions d'hybridation sur chaque zone de dépôt constituée d'oligonucléotide ON 1. On recouvre chaque zone d'une lamelle (sans que les lamelles ne se touchent). On place la lame dans une chambre d'hybridation (Corning) que l'on plonge dans un bain à 42°C pendant une heure. Les lames sont ensuite lavées avec le tampon de lavage 1 pendant 5 minutes à température ambiante ou à 50°C puis avec le tampon de lavage 2 pendant 5 minutes à 50°C. Elles sont enfin séchées sous courant d'azote.

### 2) Résultats

### 2-a) Réutilisation des dendripuces et sensibilité de la détection en fonction de la nature du tampon de dépôt, de la concentration en ON et de la nature des dendrimères

Sur les dendripuces DP1-A-G3, DP1-A-G4 et DP1-A-G5, et pour chaque concentration testée de cibles à détecter, les étapes d'hybridation, de lecture des lames et de déshybridation ont été effectuées 7 fois avec l'ON 15-mer Cy5 (200 nM), afin de démontrer le caractère réutilisable des dendripuces conformes à l'Invention, ainsi que la sensibilité et la reproductibilité des résultats obtenus. Les valeurs d'intensité de fluorescence sont regroupées dans les Tableaux II à IV ci-après ; le PMT a valeur de 600 et les dépôts ont été réalisés en triplicata pour chaque tampon :

**TABLEAU II**

| **DP1-A-G3 : APRÈS LA PREMIÈRE HYBRIDATION** | | | | | |
|---|---|---|---|---|---|
| Concentration ON 1 : 5 µM | | | Concentration ON1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 90 | 65000/200 | 0 | 100 | 65000/200 |
| 10 | 110 | 65000/200 | 10 | 110 | 65000/200 |
| 20 | 110 | 65000/200 | 20 | 140 | 65000/200 |
| 30 | 140 | 65000/200 | 30 | 140 | 65000/200 |
| 40 | 140 | 65000/200 | 40 | 150 | 65000/200 |
| 50 | 160 | 42000/200 | 50 | 160 | 50000/200 |

| **APRÈS SEPT HYBRIDATIONS** | | | | | |
|---|---|---|---|---|---|
| Concentration ON1 : 5 µM | | | Concentration ON 1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 90 | 65000/300 | 0 | 100 | 65000/350 |
| 10 | 110 | 65000/300 | 10 | 110 | 65000/350 |
| 20 | 110 | 65000/300 | 20 | 140 | 65000/350 |
| 30 | 140 | 65000/300 | 30 | 140 | 65000/350 |
| 40 | 140 | 65000/300 | 40 | 150 | 65000/350 |
| 50 | 160 | 50000/300 | 50 | 160 | 50000/350 |

**TABLEAU III**

| **DP1-A-G4 : APRÈS LA PREMIÈRE HYBRIDATION** | | | | | |
|---|---|---|---|---|---|
| Concentration ON1 : 5 µM | | | Concentration ON1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 90 | 65000/300 | 0 | 100 | 65000/300 |
| 10 | 120 | 65000/300 | 10 | 110 | 65000/300 |
| 20 | 120 | 53000/300 | 20 | 110 | 65000/300 |
| 30 | 140 | 65000/300 | 30 | 170 | 65000/300 |
| 40 | 160 | 65000/300 | 40 | 190 | 65000/300 |
| 50 | 170 | 53000/200 | 50 | 180 | 65000/200 |

| **APRÈS SEPT HYBRIDATIONS** | | | | | |
|---|---|---|---|---|---|
| Concentration ON1 : 5 µM | | | Concentration ON1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 90 | 65000/300 | 0 | 100 | 65000/350 |
| 10 | 110 | 65000/300 | 10 | 110 | ^{.}65000/350 |
| 20 | 110 | 56000/300 | 20 | 140 | 45000/350 |
| 30 | 140 | 52000/300 | 30 | 140 | 65000/350 |
| 40 | 140 | 65000/300 | 40 | 150 | 65000/350 |
| 50 | 160 | 48000/300 | 50 | 160 | 51000/350 |

**TABLEAUIV**

| **DP1-A-G5 : APRÈS LA PREMIÈRE HYBRIDATION** | | | | | |
|---|---|---|---|---|---|
| Concentration ON1 : 5 µM | | | Concentration ON 1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 100 | 65000/200 | 0 | 90 | 65000/200 |
| 10 | 110 | 65000/200 | 10 | 100 | 65000/200 |
| 20 | 130 | 65000/200 | 20 | 120 | 45000/200 |
| 30 | 160 | 38000/200 | 30 | 170 | 65000/200 |
| 40 | 170 | 65000/200 | 40 | 180 | 65000/200 |
| 50 | 180 | 47000/200 | 50 | 170 | 48000/200 |

| **APRÈS SEPT HYBRIDATIONS** | | | | | |
|---|---|---|---|---|---|
| Concentration ON1 : 5 µM | | | Concentration ON1 : 10 µM | | |
| Concentration DMSO (%) | Diamètre des dépôts (µm) | Signal/Bruit | Concentration DMSO (%) | Diamètre des dépôts (µm) | Signal/Bruit |
| 0 | 90 | 65000/500 | 0 | 100 | 65000/500 |
| 10 | 110 | 65000/500 | 10 | 110 | 65000/500 |
| 20 | 110 | 56000/500 | 20 | 140 | 45000/500 |
| 30 | 140 | 52000/500 | 30 | 140 | 65000/500 |
| 40 | 140 | 60000/500 | 40 | 150 | 62000/500 |
| 50 | 160 | 42000/500 | 50 | 160 | 60000/500 |

Ces résultats montrent que les rapports signal/bruit sont pratiquement constants entre la première et la septième hybridation. Ces résultats démontrent que les dendripuces conformes à l'Invention peuvent être réutilisées sans diminution du rapport signal/bruit.

De plus, ces résultats montrent que les rapports signal/bruit obtenus avec les différentes concentrations de tampon sont très proches et donc significatifs de la reproductibilité et de la sensibilité des mesures.

De la même manière, 10 cycles d'hybridation et de déshybridation ont été réalisés sur les dendripuces DP1-B-G4 telles que préparées ci-dessus à l'exemple 2.

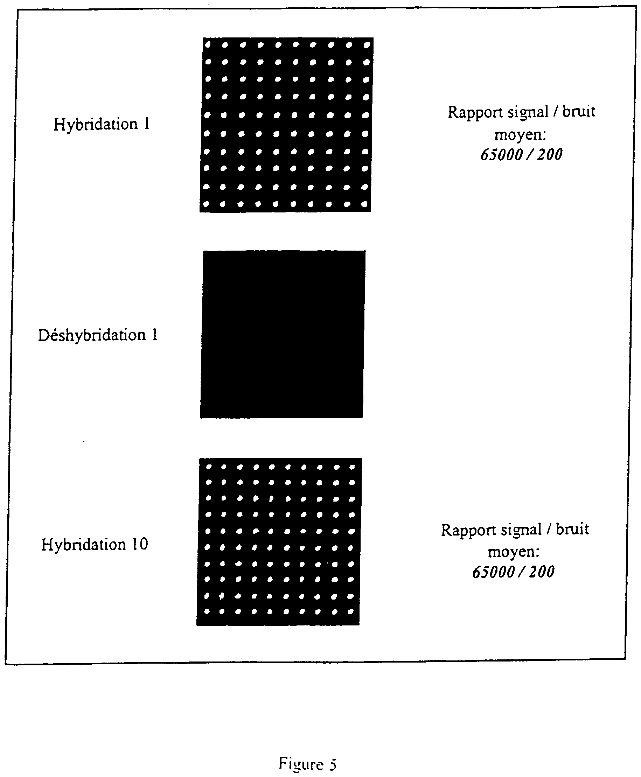

Les résultats obtenus, sous la forme d'images des signaux de fluorescence, sont représentés sur la figure 5.

Ces résultats montrent que le rapport signal/bruit après la première hybridation (65000/200) est le même après la dixième hybridation et donc significatif du caractère réutilisable des dendripuces conformes à la présente Invention.

### 2-b) Réutilisation des dendripuces et sensibilité de la détection en fonction de la concentration en ON cible et de la nature des dendrimères

L'oligonucléotide ON1 a été déposé en cinq endroits différents sur les dendripuces DP1-A-G3, DP1-A-G4 et DP1-A-G5 telles que préparées à l'exemple 3 ci-dessus.

Les solutions d'hybridation suivantes contenant des concentrations décroissantes en ON-Cy5 cible ont été utilisées afin d'évaluer la sensibilité des dendripuces conformes à l'Invention :
- SSPE 2x, SDS 0,1%, [ON- Cy5] = 200 nM
- SSPE 2x, SDS 0,1%, [ON-Cy5] = 100 nM
- SSPE 2x, SDS 0,1%, [ON-Cy5] = 20 nM
- SSPE 2x, SDS 0,1%, [ON-Cy5] = 10 nM
- SSPE 2x, SDS 0, 1 %, [ON-Cy5] = 2 nM
- SSPE 2x, SDS 0,1%, [ON-Cy5] = 1 nM

2 µl de chacune de ces solutions d'hybridation ont ensuite été déposés sur les dendripuces et laissés incuber pendant 15 minutes à température ambiante. Les dendripuces ont ensuite été lavées avec le tampon SSPE 2x, SDS 0,1% pendant 5 minutes à température ambiante avant d'être séchées sous courant d'azote ou par centrifugation. 10 cycles d'hybridation/déshybridation ont ainsi été effectués.

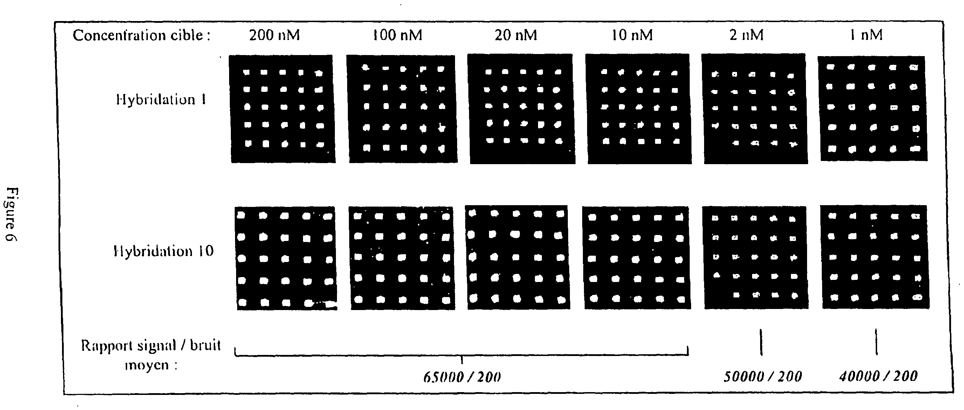

Les images obtenues sont représentées sur la figure 6.

Ces résultats montrent que pour une concentration en ON-Cy5 cible variant entre 200 nM et 10 nM, et même après 10 cycles d'hybridation/déshybridation, on obtient un rapport signal/bruit constant de 65000/200. Pour une concentration en ON-Cy5 cible inférieure, c'est-à-dire de 2nM ou de 1nM, on obtient des rapports signal/bruit respectivement de 50000/200 et de 40000/200 significatifs de la très bonne sensibilité de détection des dendripuces conformes à l'Invention et de la reproductibilité des résultats qu'elles permettent d'obtenir lorsqu'elles sont réutilisées plusieurs fois.

### 2-b) Détection de mutations

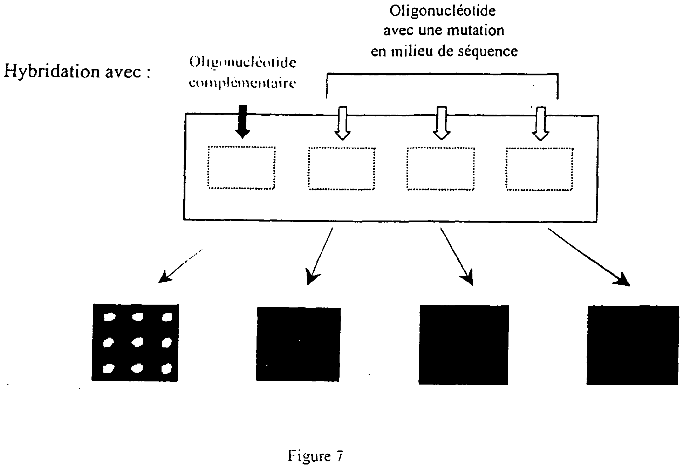

Les résultats obtenus sont représentés sur la figure 7 annexée sur laquelle on peut voir que seul l'oligonucléotide parfaitement complémentaire conduit à l'obtention d'un signal de fluorescence alors qu'aucun signal de fluorescence n'est obtenu avec les oligonucléotides contenant une mutation en milieu de chaîne. Les dendripuces conformes à l'Invention permettent donc de détecter des mutations.

### EXEMPLE 5: SYNTHÈSE D'UN DENDRIMÈRE DE GÉNÉRATION 3 A EXTRÉMITÉS THIOLS

### 1) Première étape : Synthèse d'un dendrimère de pénération 3 à extrémités NH(Me)

A une solution de dendrimère de génération 3 (coeur N₃P₃, Launay *et al*, pré-cité) à extrémités aldéhydes (650 mg) dans le dichlorométhane est ajoutée, à la seringue, un léger excès de monométhylhydrazine (224 µl). Après quatre heures d'agitation à température ambiante le milieu réactionnel est concentré sous pression réduite puis le dendrimère de génération 3 à extrémités NH(Me) est précipité en utilisant du diéthyléther (3x15 ml). La poudre blanche résultante est lavée plusieurs fois avec ce solvant avant d'être séchée sous pression réduite.

### 2) Deuxième étape : Synthèse d'un dendrimère de pénération 3 à extrémités thiols

Le dendrimère de génération 3 à extrémités NH(Me) obtenu à l'étape 1) ci-dessus (1 g) est dissous dans 2,5 ml de γ-thiobutyrolactone dans un tube de Schlenk. Le mélange est maintenu sous agitation pendant 3 jours à 50-55°C sous pression autogène. Après refroidissement, le brut réactionnel est lavé avec 3x50 ml de diéthyléther pour donner un dendrimère de génération 3 possédant 48 fonctions thiols à sa périphérie.

Ce dendrimère, qui est représenté sur la figure 3 annexée, peut ensuite être utilisé pour la fabrication des dendrilames conformes à l'Invention. Remarque : cette procédure est générale et permet d'obtenir des dendrimères à extrémités thiols de toutes les générations (Schmid et al, Chem. Eur. J., 2000, 6, 1693).

### EXEMPLE 6: PRÉPARATION DE "DENDRILAMES" FONCTIONNALISÉES PAR DES DENDRIMÈRES A EXTRÉMITÉS THIOLS

Des lames en SiO₂, SiO₂ silanisées ou SiNx sur lesquelles sont fixés des plots d'or (10 µm² à 500 µm²) sont préalablement lavées avec de l'éthanol à 95 % et séchées à l'air comprimé. Elles sont ensuite plongées pour des durées variables (15 minutes, 1 heure, 2 heures et 12 heures) dans une solution constituée de 100 µg de dendrimères de génération 3 à extrémités thiols tel que préparé ci-dessus à l'exemple 5 dissous dans 5 ml de THF distillé. Les lames sont ensuite lavées avec du THF distillé (± ultrasons) puis avec de l'éthanol avant d'être séchées à l'air comprimé. Le résultat du greffage (non représenté) est observé par microscopie à force atomique (AFM). Dans le cas des plots d'or sur support SiO₂, le greffage des dendrimères G3-thiols est non sélectif (fixation à la fois sur l'or et sur le support SiO₂) Dans le cas des supports SiO₂ silanisés ou SiNx, le greffage des dendrimères G3-thiols se fait sélectivement sur les plots d'or. On obtient ainsi des lames en silicium/or dont la surface est fonctionnalisée par des dendrimères-thiol de génération 3 (dendrilames : DL-C-G3'). Ces dendrilames peuvent ensuite être utilisées pour l'immobilisation de molécules d'intérêt.

## Revendications

1. Support solide **caractérisé par le fait qu'**il comprend au moins une surface fonctionnalisée de façon covalente par des dendrimères phosphorés possédant un noyau central qui contient au moins deux groupements fonctionnels et comportant à leur périphérie plusieurs fonctions aptes à permettre la fixation desdits dendrimères sur ladite surface ainsi que la fixation ou la synthèse *in situ* de molécules d'intérêt, lesdits dendrimères ayant une taille comprise entre 1 et 20 nm, et
**par le fait que** les dendrimères sont choisis parmi ceux qui sont constitués :
- d'une couche centrale sous la forme d'un noyau central P₀, éventuellement phosphoré, comprenant de 2 à 12 groupements fonctionnalisés,
- de n couches intermédiaires, identiques ou différentes, chacune desdites couches intermédiaires étant constituée d'unités P₁ répondant à la formule (I) suivante : 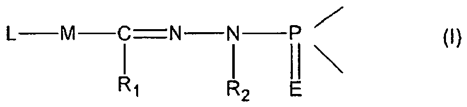 dans laquelle :
**L** est un atome d'oxygène, de phosphore, de soufre ou d'azote,
**M** représente l'un des groupes suivants :
• un groupe aromatique non substitué, di-, tri- ou tétrasubstitué par des groupes alkyles, des groupes alcoxy, des groupements insaturés du type oléfinique en C₁-C₁₂, azoïques, acétylénique, tous ces groupes pouvant incorporer ou non des atomes de phosphore, d'oxygène, d'azote, de soufre ou des halogènes, ou
• un groupe alkyle ou alcoxy comportant plusieurs substituants tels que définis lorsque M est un groupe aromatique,
**R₁ et R₂**, qui peuvent être identiques ou différents, représentent un atome d'hydrogène ou l'un des groupes suivants : alkyle, alcoxy, aryle, comportant ou non des atomes de phosphore, d'oxygène, de soufre, d'azote ou des halogènes avec R₂ étant le plus souvent différent de R₁,
**n** est un nombre entier compris entre 1 et 11,
**E** est un atome d'oxygène, de soufre ou d'azote, ledit atome d'azote pouvant être lié à un groupe alkyle, alcoxy ou aryle, tous ces groupes pouvant incorporer ou non des atomes de phosphore, d'oxygène, d'azote, de soufre ou des halogènes,
- une couche externe constituée d'unités P₂, identiques ou différentes, et répondant à la formule II suivante : 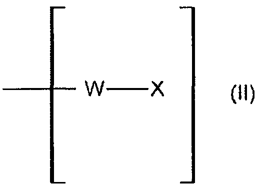 dans laquelle:
**W** représente l'un des groupes suivants : alkyle, alcoxy, aryle, tous ces groupes comportant ou non des atomes de phosphore, d'oxygène, d'azote, de soufre ou des halogènes,
**X** représente un groupement aldéhyde, thiol, amine, époxyde, acide carboxylique, alcool ou phénol.

2. Support solide selon la revendication 1, **caractérisé par le fait que** le noyau central P₀ est sélectionné dans le groupe constitué par le groupement de formule générale IIIa : 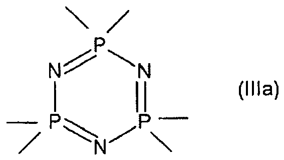 et le groupement de formule générale IIIb : 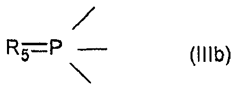 dans lequel R₅ représente un atome de soufre, d'oxygène ou d'azote.

3. Support selon la revendication 1 ou 2, **caractérisé par le fait que** les dendrimères sont choisis parmi les composés dans lesquels le groupe de formule (I) représente le groupe suivant : 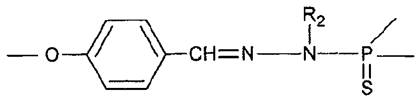 dans lequel R₂ représente un radical alkyle en C₁-C₁₂ et plus particulièrement un radical méthyle ;
et le groupe de formule (II) représente l'un des deux groupes suivants : 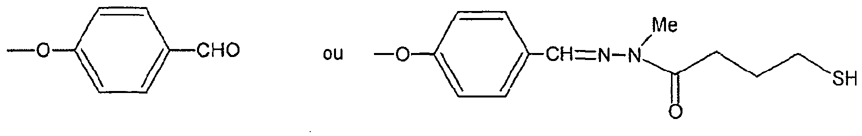 et dans lesquels le nombre de générations varie de préférence entre 1 et 6.

4. Support solide selon l'une quelconque des revendications précédentes, **caractérisé par le fait qu'**il est choisi parmi les supports comportant au moins une surface siliciée telle que les lames, les billes et les capillaires en verre, les supports en silicium, en plastique et les supports métalliques.

5. Procédé de préparation d'un support solide tel que défini à l'une quelconque des revendications 1 à 4, **caractérisé par le fait qu'**il comprend une étape de formation d'une liaison covalente entre des dendrimères phosphorés possédant un noyau central qui contient au moins deux groupements fonctionnels, lesdits dendrimères comportant à leur périphérie plusieurs fonctions aptes à permettre leur fixation sur ladite surface et la fixation ou la synthèse *in situ* de molécules d'intérêt, et la surface fonctionnalisée ou non d'un support solide pour obtenir un support solide fonctionnalisé de façon covalente par lesdits dendrimères.

6. Procédé selon la revendication 5, **caractérisé par le fait que** les dendrimères comportent à leur périphérie des fonctions permettant l'accrochage direct par l'intermédiaire d'une liaison covalente de ces derniers sur la surface non préalablement fonctionnalisée dudit support solide.

7. Procédé selon la revendication 6, **caractérisé par le fait que** les dendrimères comportent à leur périphérie des fonctions thiol et que le support solide comprend une surface d'or.

8. Procédé selon la revendication 5, dans lequel la surface du support solide utilisé ne comprend pas de fonctions compatibles avec les fonctions périphériques du dendrimère utilisé, **caractérisé par le fait qu'**il comprend les étapes suivantes :
a) la fonctionnalisation d'au moins une surface d'un support solide par des fonctions aptes à permettre la fixation de dendrimères phosphorés possédant un noyau central qui contient au moins deux groupements fonctionnels, lesdits dendrimères comportant à leur périphérie plusieurs fonctions aptes à permettre leur fixation sur ladite surface ainsi fonctionnalisée et la fixation ou la synthèse *in situ* de molécules d'intérêt ;
b) la préactivation éventuelle des fonctions du support pour obtenir une surface fonctionnalisée activée,
c) la formation d'une liaison covalente entre lesdits dendrimères et ladite surface fonctionnalisée et éventuellement activée, pour obtenir un support solide fonctionnalisé de façon covalente par lesdits dendrimères.

9. Procédé selon la revendication 8, **caractérisé par le fait que** l'étape a) de fonctionnalisation de la surface du support solide est réalisée par silanisation au moyen d'un réactif de silanisation comportant des fonctions aptes à fixer les dendrimères, telles que par exemple des groupements amine.

10. Procédé selon la revendication 8 ou 9, **caractérisé par le fait que** le réactif de silanisation est aminé.

11. Procédé selon l'une quelconque des revendications 8 à 10, **caractérisé par le fait que** l'étape de préactivation est réalisée par traitement du support à l'aide d'un agent alcalinisant, pendant une durée comprise entre 2 et 20 minutes.

12. Procédé selon l'une quelconque des revendications 5 à 11, **caractérisé par le fait que** l'étape de fixation covalente des dendrimères, consiste à :
- préparer une solution desdits dendrimères dans un solvant,
- mettre ladite solution de dendrimères en contact avec la surface éventuellement fonctionnalisée et éventuellement activée, pendant une durée comprise entre 10 minutes et 24 heures.

13. Procédé selon l'une quelconque des revendications 5 à 12, **caractérisé par le fait qu'**à l'issue de l'étape de fixation covalente des dendrimères, les supports sont rincés puis séchés.

14. Utilisation d'un support solide fonctionnalisé par des dendrimères phosphorés tel que défini à l'une quelconque des revendications 1 à 4, comme support pour l'immobilisation et/ou la synthèse *in situ* de molécules d'intérêt.

15. Utilisation selon la revendication 14, **caractérisée par le fait que** les molécules d'intérêt sont des molécules d'acide nucléique, des lipides, des protéines ou leurs partenaires moléculaires.

16. Biopuce ou dendripuce **caractérisée par le fait qu'**elle est constituée d'un support solide comportant au moins une surface fonctionnalisée par des dendrimères phosphorés et tels que définis à l'une quelconque des revendications 1 à 4, sur lesquels sont fixées, de façon covalente, des molécules d'intérêt.

17. Biopuce selon la revendication 16, **caractérisée par le fait qu'**elle est réutilisable.

18. Procédé de préparation d'une biopuce telle que définie à la revendication 16 ou 17, **caractérisé par le fait qu'**il consiste à mettre en contact un support solide ayant au moins une surface fonctionnalisée par des dendrimères phosphorés et comportant à leur périphérie des fonctions aptes à permettre la fixation covalente de molécules d'intérêt avec une solution tampon renfermant des molécules d'intérêt préalablement fonctionnalisées par, ou comportant déjà, un ou plusieurs groupements aptes à former une liaison covalente avec lesdites fonctions périphériques des dendrimères.

19. Procédé selon la revendication 18, **caractérisé par le fait que** les fonctions périphériques des dendrimères utilisés sont des fonctions aldéhydes, et que les molécules d'intérêt sont préalablement fonctionnalisées par, ou contiennent déjà, une ou plusieurs fonctions amine, ou sont préalablement fonctionnalisées par une ou plusieurs fonctions oxyamine (-ONH₂) ou hydrazine (-NH-NH₂).

20. Procédé selon la revendication 19, **caractérisé par le fait que** les molécules d'intérêt sont préalablement fonctionnalisées par, ou contiennent déjà, une ou plusieurs fonctions amine et que l'étape de fixation des molécules d'intérêt est suivie d'une étape de réduction des fonctions imines.

21. Procédé selon la revendication 18, **caractérisé par le fait que** les fonctions périphériques des dendrimères utilisés sont des fonctions thiol, et que les molécules d'intérêt sont préalablement fonctionnalisées par, ou contiennent déjà, une ou plusieurs fonctions thiol ou sont préalablement fonctionnalisées par une ou plusieurs fonctions iodoacétamido (-NHCO-CH₂-I).

22. Procédé selon la revendication 18, **caractérisé par le fait que** les fonctions périphériques des dendrimères utilisés sont des fonctions amines, et que les molécules d'intérêt sont préalablement fonctionnalisées par, ou contiennent déjà, une ou plusieurs fonctions aldéhyde, α-oxoaldéhyde, -COOR, -NCS ou -NHS.

23. Procédé selon la revendication 18, **caractérisé par le fait que** les fonctions périphériques des dendrimères utilisés sont des fonctions époxyde, et que les molécules d'intérêt sont préalablement fonctionnalisées par, ou contiennent déjà, une ou plusieurs fonctions amine.

24. Procédé selon l'une quelconque des revendications 18 à 23, **caractérisé par le fait que** la réaction de fixation covalente des molécules d'intérêt sur les dendrilames est réalisée à une température comprise entre 4 et 50°C, pendant une durée comprise entre 2 et 24 heures.

25. Utilisation d'une dendripuce selon la revendication 16 ou 17 comme puces à ADN, à peptides, à polypeptides ou à protéines.

## Claims

1. Solid support, **characterized in that** it comprises at least one surface covalently functionalized by phosphorus-containing dendrimers having a central nucleus which contains at least two functional groups and comprising at their periphery several functions which are capable of allowing the binding of the said dendrimers to the said surface as well as the binding or in situ synthesis of molecules of interest, the said dendrimers having a size of between 1 and 20 nm, and
**in that** the dendrimers are chosen from those which are made up of:
- a central layer in the form of a central nucleus P₀, optionally containing phosphorus, comprising 2 to 12 functionalized groups,
- n intermediate layers which are identical or different, each of the said intermediate layers being made up of P₁ units corresponding to the following formula (I): 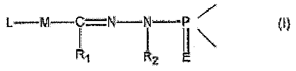
in which:
**L** is an oxygen, phosphorus, sulfur or nitrogen atom,
**M** represents one of the following groups:
• an aromatic group which is unsubstituted or di-, tri- or tetrasubstituted by alkyl groups, alkoxy groups or unsaturated groups of the C₁-C₁₂-olefin, azo or acetylene type, it being possible for all these groups optionally to incorporate phosphorus, oxygen, nitrogen, sulfur or halogen atoms, or
• an alkyl or alkoxy group containing several substituents as defined for M as an aromatic group,
**R₁ and R₂**, which can be identical or different, represent a hydrogen atom or one of the following groups: alkyl, alkoxy or aryl, optionally containing phosphorus, oxygen, sulfur, nitrogen or halogen atoms, where R₂ is most often different from R₁,
**n** is an integer between 1 and 11,
2. The method according to claim 1 wherein said source communications channel (22) is overloaded with multicast data from a plurality of multicast data sources. is an oxygen, sulfur or nitrogen atom, it being possible for the said nitrogen atom to be bonded to an alkyl, alkoxy or aryl group, it being possible for all these groups optionally to incorporate phosphorus, oxygen, nitrogen, sulfur or halogen atoms,
- an external layer made up of P₂ units which are identical or different and correspond to the following formula II: 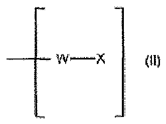
in which:
**W** represnts one of the following groups: alkyl, alkoxy or aryl, all these groups optionally containing phosphorus, oxygen, nitrogen, sulfur or halogen atoms,
**X** represents an aldehyde, thiol, amine, epoxide, carboxylic acid, alcohol or phenol groups.

2. Solid support according to claim 1, **characterized in that** the central nucleus P₀ is selected from the group made up of a group of the general formula IIIa: 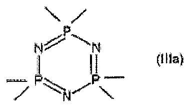 and a group of the general formula IIIb: 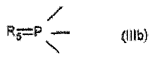 in which R₅ represents a sulfur, oxygen or nitrogen atom.

3. Support according to claim 1 or 2, **characterized in that** the dendrimers are chosen from the compounds in which the group of the formula (I) represents the following group: 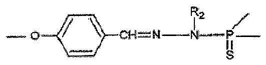 in which R₂ represents a C₁-C₁₂-alkyl radical, and more particularly a methyl radical;
and the group of the formula (II) represents one of the following two groups: 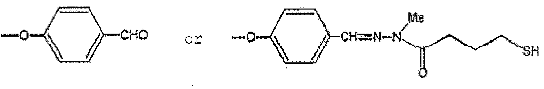 and in which the number of generations preferably varies between 1 and 6.

4. Solid support according to any one of the preceding claims, **characterized in that** it is chosen from supports comprising at least one siliceous surface, such as glass slides, beads and capillaries, supports of silicon or plastic and metallic supports.

5. Process for the preparation of a solid support as defined in any one of claims 1 to 4, **characterized in that** it comprises a step of formation of a covalent bond between the phosphorus-containing dendrimers having a central nucleus which contains at least two functional groups, the said dendrimers comprising at their periphery several functions which are capable of allowing binding thereof to the said surface and binding or in situ synthesis of molecules of interest, and the optionally functionalized surface of a solid support to obtain a solid support covalently functionalized by the said dendrimers.

6. Process according to claim 5, **characterized in that** the dendrimers comprise at their periphery functions allowing direct attachment, via a covalent bond, of the latter on to the surface, which has not be functionalized beforehand, of the said solid support.

7. Process according to claim 6, **characterized in that** the dendrimers comprise at their periphery thiol functions, and the solid support comprises a surface of gold.

8. Process according to claim 5, in which the surface of the solid support used does not comprise functions compatible with the peripheral functions of the dendrimer used, **characterized in that** it comprises the following steps:
a) functionalization of at least one surface of a solid support by functions which are capable of allowing the binding of phosphorus-containing dendrimers having a central nucleus which contains at least two functional groups, the said dendrimers comprising at their periphery several functions which are capable of allowing binding thereof to the said surface functionalized in this way and the binding or *in situ* synthesis of molecules of interest;
b) optional preactivation of functions of the support to obtain an activated functionalized surface,
c) formation of a covalent bond between the said dendrimers and the said functionalized and optionally activated surface to obtain, a solid support covalently functionalized by the said dendrimers.

9. Process according to claim 8, **characterized in that** step a) of functionalization of the surface of the solid support is carried out by silanization by means of a silanization reagent comprising functions which are capable of binding the dendrimers, such as, for example, amine groups.

10. Process according to claim 8 or 9, **characterized in that** the silanization reagent is aminated.

11. Process according to any one of claims 8 to 10, **characterized in that** the preactivation step is carried out by treatment of the support with the aid of an alkalizing agent for a period of between 2 and 20 minutes.

12. Process according to any one of claims 5 to 11, **characterized in that** the step of covalent binding of the dendrimers consists of:
- preparation of a solution of the said dendrimers in a solvent,
- bringing the said solution of dendrimers into contact with the optionally functionalized and optionally activated surface for a period of between 10 minutes and 24 hours.

13. Process according to any one of claims 5 to 12, **characterized in that** at the end of the step of covalent binding of the dendrimers, the supports are rinsed and then dried.

14. Use of a solid support functionalized by phosphorus-containing dendrimers as defined in any one of claims 1 to 4 as a support for immobilization and/or *in situ* synthesis of molecules of interest.

15. Use according to claim 14, **characterized in that** the molecules of interest are nucleic acid molecules, lipids, proteins or molecular partners thereof.

16. Biochip or dendrichip, **characterized in that** it is made up of a solid support comprising at least one surface functionalized by phosphorus-containing dendrimers and as defined in any one of claims 1 to 4, on to which are covalently bound molecules of interest.

17. Biochip according to claim 16, **characterized in that** it is reusable.

18. Process for the preparation of a biochip as defined in claim 16 or 17, **characterized in that** it consists of bringing a solid support having at least one surface functionalized by phosphorus-containing dendrimers and comprising at their periphery functions which are capable of allowing covalent binding of molecules of interest into contact with a buffer solution containing the molecules of interest which have been functionalized beforehand by, or already comprising, one or more groups which are capable of forming a covalent bond with the said peripheral functions, of the dendrimers.

19. Process according to claim 18, **characterized in that** the peripheral functions of the dendrimers used are aldehyde functions, and the molecules of interest have been functionalized beforehand by, or already contain, one or more amine functions, or have been functionalized beforehand by one or more oxyamine (-ONH₂) or hydrazine (-NH-NH₂) functions.

20. Process according to claim 19, **characterized in that** the molecules of interest have been functionalized beforehand by, or already contain, one or more amine functions, and the step of binding of the molecules of interest is followed by a step of reduction of the imine functions.

21. Process according to claim 18, **characterized in that** the peripheral functions of the dendrimers used are thiol functions, and the molecules of interest have been functionalized beforehand by, or already contain, one or more thiol functions or have been functionalized beforehand by one or more iodoacetamido (-NHCO-CH₂-I) functions.

22. Process according to claim 18, **characterized in that** the peripheral functions of the dendrimers used are amine functions, and the molecules of interest have been functionalized beforehand by, or already contain, one or more aldehyde, α-oxoaldehyde, -COOR, -NCS or -NHS functions.

23. Process according to claim 18, **characterized in that** the peripheral functions of the dendrimers used are epoxide functions, and the molecules of interest have been functionalized beforehand by, or already contain, one or more amine functions.

24. Process according to any one of claims 18 to 23, **characterized in that** the reaction of covalent binding of the molecules of interest on to the dendrislides is carried out at a temperature of between 4 and 50 °C, over a period of between 2 and 24 hours.

25. Use of a dendrichip according to claim 16 or 17 as DNA, peptide, polypeptide or protein chips.

## Patentansprüche

1. Fester Träger, **dadurch gekennzeichnet, dass** er wenigstens eine Oberfläche umfasst, die in kovalenter Art durch phosphorhaltige Dendrimere funktionalisiert ist, welche einen zentralen Kern, der wenigstens zwei funktionelle Gruppierungen enthält, besitzen und an ihrer Peripherie mehrere Funktionen aufweisen, die geeignet sind, die Fixierung der Dendrimere auf der genannten Oberfläche sowie die Fixierung oder die *in-situ-*Synthese von Molekülen von Interesse zu ermöglichen, wobei die Dendrimere eine Größe von zwischen 1 und 20 nm haben, und
dass die Dendrimere aus denen ausgewählt sind, die bestehen aus:
- einer zentralen Schicht in der Form eines zentralen Kerns P₀, gegebenenfalls phosphorhaltig, der 2 bis 12 funtionalisierte Gruppierungen umfasst,
- n Zwischenschichten, die identisch oder unterschiedlich sind, wobei jede der Zwischenschichten aus Einheiten P₁ besteht, die der folgenden Formel (I) entsprechen: 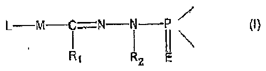 in der:
**L** ein Sauerstoff-, Phosphor-, Schwefel- oder Stickstoffatom ist,
**M** eine der folgenden Gruppen darstellt:
• eine aromatische Gruppe, die nicht substituiert, di-, tri- oder tetrasubstituiert ist mit Alkylgruppen, Alkoxygruppen, ungesättigten C₁-C₁₂-Gruppierungen des olefinischen Typs, Azotyps, Acetylentyps, wobei alle diese Gruppen Phosphor-, Sauerstoff-, Stickstoff-, Schwefel-Atome oder Halogene eingebaut haben können oder nicht, oder
• eine Alkyl- oder Alkoxygruppe, die mehrere Substituenten umfasst, wie sie definiert sind, wenn M eine aromatische Gruppe ist,
R₁ und R₂, die identisch oder unterschiedlich seinkönnen, ein Wasserstoffatom oder eine der folgenden Gruppen darstellen: Alkyl, Alkoxy, Aryl, umfassend Phosphor-, Sauerstoff-, Schwefel-, Stickstoffatome oder Halogene, wobei R₂ meist von R₁ verschieden ist,
**n** eine ganze Zahl zwischen 1 und 11 ist,
**E** ein Sauerstoff-, Schwefel- oder Stickstoffatom ist, wobei das Stickstoffatom an eine Alkyl-, Alkoxy- oder Arylgruppe gebunden sein kann, wobei alle diese Gruppen Phosphor-, Sauerstoff-, Stickstoff-, Schwefelatome oder Halogene eingearbeitet haben können,
- eine äußere Schicht, die aus Einheiten P₂ besteht, die identisch oder unterschiedlich sind und der folgenden Formel II entsprechen: 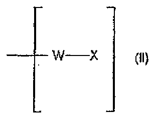 in der:
**W** eine der folgenden Gruppen darstellt: Alkyl, Alkoxy, Aryl, wobei alle diese Gruppen Phosphor-, Sauerstoff- , Stickstoff-, Schwefelatome oder Halogene tragen oder nicht,
**X** eine Aldehyd-, Thiol-, Amin-, Epcxid-, Carbonsäure-, Alkohol- oder Phenolgruppierung darstellt.

2. Fester Träger gemäß Anspruch 1, **dadurch gekennzeichnet, dass** der zentrale Kern P₀ aus der Gruppe ausgewählt ist, die aus der Gruppierung der allgemeinen Formel IIIa: 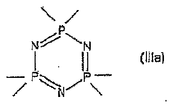 und der Gruppierung der allgemeinen Formel IIIb 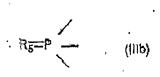 in der R₅ ein Schwefel-, Sauerstoff- oder Stickstoffatom darstellt, besteht.

3. Träger gemäß Anspruch 1 oder 2, **dadurch gekennzeichnet, dass** die Dendrimere aus den Verbindungen ausgewählt sind, in denen die Gruppe der Formel (I) die folgende Gruppe darstellt: 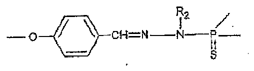 in der R₂ einen C₁-C₁₂-Alkylrest und insbesondere einen Methylrest darszellt;
und die Gruppe der Formel (II) eine der folgenden zwei Gruppen darstellt: 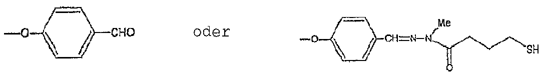 und in denen die Generationenzahl vorzugsweise zwischen 1 und 6 variiert.

4. Fester Träger gemäß einem der vorangehenden Ansprüche, **dadurch gekennzeichnet, dass** er unter den Trägern ausgewählt ist, die wenigstens eine silizierte Oberfläche umfassen wie Plättchen, Perlen und Kapillaren aus Glas, Träger aus Silicium, Kunststoff und Metallträger.

5. Verfahren zur Herstellung eines festen Trägers, wie er in einem der Ansprüche 1 bis 4 definiert ist, **dadurch gekennzeichnet, dass** es eine Stufe der Bildung einer kovalenten Bindung zwischen den phosphorhaltigen Dendrimeren, die einen zentralen Kern besitzen, der wenigstens zwei funktionelle Gruppierungen enthält, wobei die Dendrimere an ihrer Peripherie mehrere Funktionen aufweisen, die geeignet sind, ihre Fixierung auf der genannten Oberfläche und die Fixierung oder die *in-situ*-synthese von Molekülen von Interesse zu ermöglichen, und der funktionalisierten oder nicht funktionalisiorten Oberfläche eines festen Trägers unter Erhalt eines festen Trägers, der in kovalenter Art durch die Dendrimere funktionalisiert ist, umfasst.

6. Verfahren gemäß Anspruch 5, **dadurch gekennzeichnet, dass** die Dendrimere an ihrer Peripherie Funktionen aufweisen, die die direkte Verankerung mittels kovalenter Bindung dieser letztgenannten auf der vorher nicht funktionalisierten Oberfläche des festen Trägers ermöglichen.

7. Verfahren gemäß Anspruch 6, **dadurch gekennzeichnet, dass** die Dendrimere an ihrer Peripherie Thiolfunktionen aufweisen und dass der feste Träger eine Goldoberfläche umfasst.

8. Verfahren gemäß Anspruch 5, in dem die Oberfläche des verwendeten festen Trägers keine Funktionen umfasst, die mit den peripheren Funktionen des verwendeten Dendrimers kompatibel sind, **dadurch gekennzeichnet, dass** es die folgenden Stufen umfasst:
a) Funktionalisierung wenigstens einer Oberfläche eines festen Trägers durch Funktionen, die geeignet sind, die Fixierung von phosphorhaltigen Dendrimeren, die einen zentralen Kern besitzen, der wenigstens zwei funktionelle Gruppierungen enthält, zu erlauben, wobei die Dendrimere an ihrer Peripherie mehrere Funktionen aufweisen, die geeignet sind, ihre Fixierung auf der so funktionalisierten Oberfläche und die Fixierung oder die *in-situ-*Synthese von Molekülen von Interesse zu ermöglichen;
b) potentielle Voraktivierung der Funktionen des Trägers unter Erhalt einer funktionalisierten aktivierten Oberfläche,
c) Bildung einer kovalenten Bindung zwischen den Dendrimeren und der funktionalisierten und gegebenenfalls aktivierten Oberfläche unter Erhalt eines festen Trägers, der in kovalenter Art durch die Dendrimere funktionalisiert ist.

9. Verfahren gemäß Anspruch 8, **dadurch gekennzeichnet, dass** die Stufe a) Funktionalisierung der Oberfläche des festen Trägers durch Silanisierung mit Hilfe eines Silanisierungsreagenzes, das Funktionen trägt, die geeignet sind, die Dendrimere zu fixieren, wie zum Beispiel Amingruppierungen, durchgeführt wird.

10. Verfahren gemäß Anspruch 8 oder 9, **dadurch gekennzeichnet, dass** das Silanisierungsreagenz aminiert ist.

11. Verfahren gemäß einem der Ansprüche 8 bis 10, **dadurch gekennzeichnet, dass** die Stufe der Voraktivierung durch Behandlung des Trägers mittels eines Alkalinisierungsmittels während einer Dauer von zwischen 2 und 20 Minuten durchgeführt wird.

12. Verfahren gemäß einem der Ansprüche 5 bis 11, **dadurch gekennzeichnet, dass** die Stufe der kovalenten Fixierung der Dendrimere aus:
- Herstellen einer Lösung der Dendrimere in einem Lösungsmittel,
- in Kontakt bringen der Dendrimerenlösung mit der gegebenenfalls funktionalisierten und gegebenenfalls aktivierten Oberfläche während einer Dauer, die zwischen 10 Minuten und 24 Stunden liegt,
besteht.

13. Verfahren gemäß einem der Ansprüche 5 bis 12, **dadurch gekennzeichnet, dass** die Träger nach der Stufe der kovalenten Fixierung der Dendrimere gespült, danach getrocknet werden.

14. Verwendung eines festen Trägers, der durch phosphorhaltige Dendrimere funktionalisiert ist, wie er in einem der Ansprüche 1 bis 14 definiert ist, als Träger für die Immobilisierung und/oder die *in-situ*-Synthese von Molekülen von Interesse.

15. Verwendung gemäß Anspruch 14, **dadurch gekennzeichnet, dass** die Moleküle von Interesse Nukleinsäuremoleküle, Lipide, Proteine oder ihre molekularen Partner sind.

16. Biochip oder Dendrichip, **dadurch gekennzeichnet, dass** er aus einem festen Träger, der wenigstens eine Oberfläche umfasst, die durch Dendrimere funktionalisiert ist, welche phosphorhaltig sind und wie in einem der Ansprüche 1 bis 4 definiert sind, an denen in kovalenter Art Moleküle von Interesse fixiert sind, besteht.

17. Biochip gemäß Anspruch 16, **dadurch gekennzeichnet, dass** er wiederverwendbar ist.

18. Verfahren zur Herstellung eines Biochips, wie er in Anspruch 16 oder 17 definiert ist, **dadurch gekennzeichnet, dass** es aus einem in Kontakt bringen eines festen Trägers, der wenigstens eine Oberfläche hat, die durch Dendrimere funkticnalisiert ist, welche phosphorhaltig sind und an ihrer Peripherie Funktionen aufweisen, die geeignet sind, die kovalente Fixierung von Molekülen von Interesse zu ermöglichen, mit einer Pufferlösung, die Moleküle von Intersse umfasst, welche vorher durch eine oder mehrere Gruppen funktionalisiert wurden oder diese bereits tragen, die geeignet sind, eine kovalente Bindung mit den peripheren Funktionen der Dendrimere zu bilden, besteht.

19. Verfahren gemäß Anspruch 18, **dadurch gekennzeichnet, dass** die peripheren Funktionen der verwendeten Dendrimere Aldehydfunktionen sind und dass die Moleküle von Interesse vorher durch eine oder mehrere Aminfunktionen funktionalisiert wurden oder diese bereits enthalten oder vorher durch eine oder mehrere Oxyamin(-ONH₂)- oder Hydrazin(-NH-NH₂)-Funktionen funktionalisiert wurden.

20. Verfahren gemäß Anspruch 19, **dadurch gekennzeichnet, dass** die Moleküle von Interesse vorher durch eine oder mehrere Aminfunktionen funktionalisiert wurden oder diese bereits enthalten und dass auf die Stufe der Fixierung der Moleküle von Interesse eine Stufe der Reduktion der Iminfunktionen folgt.

21. Verfahren gemäß Anspruch 18, **dadurch gekennzeichnet, dass** die peripheren Funktionen der verwendeten Dendrimere Thiolfunktionen sind und dass die Moleküle von Interesse vorher durch eine oder mehrere Thiolfunktionen funktionalisiert wurden oder diese bereits enthalten oder vorher durch eine oder mehrere Jodacedamido (-NHCO-CH₂-I) -Funktionen funktionalisiert wurden.

22. Verfahren gemäß Anspruch 18, **dadurch gekennzeichnet, dass** die peripheren Funktionen der verwendeten Dendrimere Aminfunktionen sind und dass die Moleküle von Interesse vorher durch eine oder mehrere Aldehyd-, α-Oxoaldehyd-, -COOR-, NCS- oder -NHS-Funktionen funktionalisiert wurden oder diese bereits enthalten.

23. Verfahren gemäß Anspruch 18, **dadurch gekennzeichnet, dass** die peripheren Funktionen der verwendeten Dendrimere Epoxidfunktionen sind und dass die Moleküle von Interesse vorher durch eine oder mehrere Aminfunktionen funktionalisiert wurden oder diese bereits enthalten.

24. Verfahren gemäß einem der Ansprüche 18 bis 23, **dadurch gekennzeichnet, dass** die Reaktion der kovalenten Fixierung der Moleküle von Interesse auf den Dendriplättchen bei einer Temperatur, die zwischen 4 und 50°C liegt, während einer Dauer, die zwischen 2 und 24 Stunden liegt, durchgeführt wird.

25. Verwendung eines Dendrichips gemäß Anspruch 16 oder 17 als DNA-Chips, Peptid-Chips, Polypeptid-Chips oder Protein-Chips.
